# EUROPEAN PATENT APPLICATION

(11) **EP 2 823 850 A1**
(43) Date of publication of application: **14.01.2015**
(21) Application number: 12870619.9
(22) Date of filing: 25.05.2012
(51) Int. Cl.: A61M 37/00, A61K 47/32

(54) **POLYMER MICRO-NEEDLE ARRAY CHIP, PREPARATION PROCESS AND USE THEREOF**

(30) Priority: 06.03.2012 CN 201210057409
(71) Applicant: Technical Institute of Physics and Chemistry of the Chinese Academy of Sciences, Haidian District Beijing 100190 (CN)
(72) Inventor: WU, Feipeng, Beijing 100190 (CN); MIAO, Yuanhua, Beijing 100190 (CN)
(74) Representative: Serjeants LLP
(86) International application number: PCT/CN2012/000726
(87) International publication number: WO 2013/131215

(57) **Abstract**

Provided is a polymer micro-needle array chip comprising a micro-needle array and a substrate on which the micro-needle array is placed; a polyacrylamide polymer with a molecular weight of 1.0x10⁴-2.0x10⁵, Vickers hardness of 150-600 HV and impact strength of 5-30 J/m is used as the material of the micro-needle array. The polymer micro-needle array chip has high mechanical strength and a sharp needle tip; and it can easily be dissolved or can swell on contact with a water-containing environment, which helps the drug to be released slowly in the skin.

## Description

### FIELD OF THE INVENTION

The present invention relates to a polymer micro-needle array chip, and preparation process and use thereof, which belongs to the field of biomedical materials and micromachining.

### BACKGROUND OF THE INVENTION

Transdermal drug delivery refers to an administration method that allows a drug to penetrate through skin at a certain speed, enter systemic circulation via capillary vessels, and take effect. Since the blockage of the outmost layer of human skin called stratum corneum has a thickness of about 30-50 µm, traditional transdermal administration method is only suitable for high fat soluble drugs of which the molecular weight is less than 500 Dalton. In order to increase the transdermal permeability of the macromolecular drugs, such as polypeptides, proteins, vaccines, scientists put forward the concept of "micro-needle patch" administration in 1970s: stratum corneum of skin was pierced by micro-needle or micro-needle array prepared using microfabrication technology to produce micron level physical channel, which make the macromolecular drugs penetrate into deep skin tissue. Micro-needle transdermal drug delivery can avoid the interference of the first pass effect in liver and the inactivated effect in stomach, and trauma is slightly small that almost does not feel pain. Therefore, micro-needle transdermal drug delivery has a wide prospect of application.

Because of the limitation of science and technology condition, the experimental study on a patch for micro-needle transdermal drug delivery was not reported until 1998 by the team of professor Prausnitz in US (Journal of Pharmaceutical Sciences, 1998, 87, 922-925), the experimental results show that by using micro-needle transdermal drug delivery, the transdermal permeability speed of model drugs, namely calcein, can be increased by four orders of magnitude than using the traditional transdermal drug delivery method. Since then administration method of micro-needle transdermal drug delivery attracts more and more scientists to launch relevant research, various kinds of patches for micro-needle transdermal drug delivery have been developed one after another.

The core element of patch for micro-needle transdermal drug delivery is micro-needle array chip, comprising regular arranging micro-needle array and substrate supporting micro-needle array, which has the characteristic of good biocompatibility and high security. Micro-needle array chip was mainly prepared from monocrystal silicon or silicon dioxide and other semiconductor materials using the method of lithography combining electrochemistry corrosion in the early (US5879326, US6503231). Semiconductor micro-needle has good biocompatibility, high hardness, and is easy to pierce skin, but it is brittle and cannot be degraded if the broken remains in the body. Monocrystal silicon micro-needle array chip itself could not store drugs, complicated storage and sustained release system was needed to design and prepare when preparing patch for micro-needle transdermal delivery (CN 102039000A, CN102018655A), so complex processing technology and high costs limited its application in clinical. Metal micro-needle array chip usually prepared using titanium, nickel alloy and other stainless steel through laser and electrical discharge machining technology and other precision micro-processing method or lithography combining electrochemistry corrosion and other method, appeared a bit late than semiconductor micro-needle array chip (CN 100402107C, CN 1415385A, CN 1562402A, CN 101254326A, CN101507857A, CN 101829396A, CN 101912663A). Metal micro-needle has good biological security and needle tip is easy to pierce skin without broken. But metal micro-needle array chip itself can't store drugs, storage and sustained release system are needed to attach when preparing correlative micro-needle patch, so it does not have wide application in clinical at present. As good biocompatibility, degradation in the body, and high security, polymer micro-needle appeared in about 2004 and developed fast. Nowadays, polymer micro-needle was mainly prepared using the material such as polymethyl methacrylate, polylactic acid, polyglycolic acid, polyglycolic acid, vinyl pyrrolidone, polydioxanone and their copolymer through template method (US 6312612, US6451240, US 20020082543, WO 2009048607, CN 100513145, CN 102000020A, CN 1415385A, CN 101072668A, CN 101254326A, CN 102026910A). The needle body itself of polymer micro-needle can wrap drugs without designing complex drug storage system, preparation process is relatively simple, and mass production can be conducted more economically. At present the disadvantages of polymer micro-needle are as follows: on the one hand its mechanical strength is not enough to pierce skin, on the other hand most of the polymer material for preparing micro-needle are insoluble in water, and need to be processed such as pouring, moulding, in molten state at high temperature,, which causes the temperature sensitive drugs, such as protein and polypeptide, lose activity.

The polyacrylamide polymer used as medical material for a long history has the characteristic of high security, and it is mainly used as human filling or repairing materials in cosmetology or curing human body injury (GB 4746551, GB2164343A, DE 1594389, CN 94195147, CN 1450118A). The aqueous solution of polyacrylamides polymer having suitable molecular weight or a group of proper molecular weight ratio still has the mobility and cannot form gelatin in high mass fraction (not less than 50%), so polymer micro-needle array chip can be prepared through the template method.

Based on the above science and technology problems, polymer micro-needle array chip, whose needle body wrapping bioactive substances or drugs, was prepared using polyacrylamides polymer meeting the medical standards creatively through template method by the inventor. A safe and reliable polymer micro-needle patch for transdermal drug delivery is prepared on the above basis, which is simple to be operated and convenient to be used.

### SUMMARY OF THE INVENTION

The first technical problem to be solved by the present invention is to provide a polymer micro-needle array chip. The micro-needle of the polymer micro-needle array chip has a high mechanical strength and a sharp needle tip, so the stratum corneum of the skin can be easily pierced; the preparation method avoids a high-temperature processing step, and is in favor of maintaining the activities of biomacromolecules drugs comprising polypeptides, proteins and the like; the polymer micro-needle array chip can easily dissolve or swell on contact with a water-containing environment, which helps the drug to be released slowly in the skin.

The second technical problem to be solved by the present invention is to provide a method for preparing the polymer micro-needle array chip.

The third technical problem to be solved by the present invention is to provide an application of the polymer micro-needle array chip in a patch for transdermal drug delivery.

To solve the above first technical problem, a technical solution provided by the present invention is as follows:

A polymer micro-needle array chip, comprising a substrate and a micro-needle array standing thereon; the matrix material of the micro-needle array of the polymer micro-needle array chip is polyacrylamides polymer.

Furthermore, the polyacrylamides polymer is polymerized from acrylamide monomer. The reaction equation is as follows:

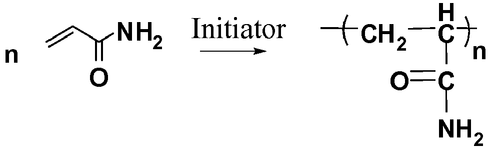

Furthermore, the molecular weight of the polyacrylamides polymer is in range of 1.0×10⁴-2.0×10⁵. The polyacrylamides polymer has a good solubility in water, which can be mixed with water to obtain an aqueous solution with the polymer in amount of 1- 80 mass%.

Furthermore, the Vickers hardness of the polyacrylamides polymer is 150-600 HV. When the polymer is under the condition of the above hardness, the micro-needle of the polymer micro-needle array chip has a high mechanical strength and a sharp needle point, so the stratum corneum of the skin can be easily pierced.

Furthermore, the impact strength of the polyacrylamides polymer is in range of 5-30 J/M. When the polymer is under the above impact strength, the micro-needle of the polymer micro-needle array chip is not easy to be broken off.

Furthermore, a square sheet prepared by the polyacrylamides polymer and having a thickness of 2 mm and sides of length 1cm, dissolves at least 50 vol% after immersing in a still physiological saline for about 6 hours.

Furthermore, the polyacrylamides polymer is mixed with bioactive substances or drugs, the bioactive substances or drugs being present in the mixture in amount of 0.1-50 mass%; preferably, 10-20 mass%. The mass percentage of the bioactive substances or drugs in the mixture can be adjusted according to the dose required and the spatial features of the resultant micro-needle array chip.

Furthermore, the bioactive substances or drugs is one or more selected from the group consisting of vaccines, polypeptides, proteins, polysaccharides, nucleic acids, hormones, anti-cancer drugs, genetic engineering drugs, natural product drugs, the traditional Chinese medicine or nutrients.

Furthermore, the amount of residual acrylamide monomer in the polyacrylamides polymer is not more than 0.5 ppm, which meets the medical standards prescribed by the World Health Organization.

Furthermore, the micro-needle array comprises at least two micro-needles; the micro-needle comprising a needle head and a needle bar; the needle bar being the body of the micro-needle with one end fixed on the substrate; the needle head being at the top of the micro-needle in any tip-like shape.

Preferably, the diameter of the largest cross-sectional circle or circumcircle of the micro-needle is in range of 50-1000 µm; the length of the micro-needle is in range of 100-5000 µm.

Furthermore, the thickness of the substrate is in range of 50-5000 µm.

Furthermore, the substrate comprises a substrate film and a substrate body; the substrate film connected to the micro-needle array with thickness less than 50 µm.

Preferably, the substrate is made of polyacrylamides polymer.

Preferably, the substrate is made of a mixture comprising polyacrylamides polymer and bioactive substances or drugs, the bioactive substances or drugs being present in the mixture in amount of 0.1-50 mass%; preferably, 10-20 mass%.

Preferably, the micro-needle array and the substrate film is made of polyacrylamides polymer, and the substrate body is a combination of one or more layers selected from the group consisting of polylactic acid, polyethylene, polypropylene, poly(butylene succinate), rubber, latex, glass and metal thermoplastic composite materials, respectively.

Preferably, the substrate is made of a mixture comprising polyacrylamides polymer and bioactive substances or drugs, the bioactive substances or drugs being present in the mixture in amount of 0.1-50 mass%; preferably, 10-20 mass%; and the substrate body is a combination of one or more layers selected from the group consisting of polylactic acid, polyethylene, polypropylene, poly(butylene succinate), rubber, latex, glass and metal thermoplastic composite materials, respectively.

Furthermore, the present invention provides a synthetic method for preparing the polyacrylamides polymer, and the synthetic reaction system mainly comprises alcohols-based organic solvent, water, acrylamide monomer and initiator; during the reaction, introducing the high purity nitrogen continuously into the reaction system , stirring rising the temperature to and holding at the target temperature, during the reaction; removing the acrylamide monomer from the reaction product and drying to obtain the polyacrylamides polymer, after the reaction. The above synthetic method has a mild condition, is simple and easy to practice, and has a high yield. The polymer prepared meets the medical standards prescribed by World Health Organization.

Specifically, a synthesis of polyacrylamide polymer comprises the following steps:

S-1, adding an organic solvent, water and acrylamide monomer in prescribed amounts into a reactor equipped with a stirring device;

The organic solvent comprises mainly alcohols, and secondly ketones; the alcohols being one or more selected from the group consisting of methanol, ethanol, n-propanol, isopropanol, n-butanol; preferably, the alcoholic solvent being present in the reaction system in amount of not less than 60 vol%; the ketonic organic solvent being one or more selected from the group consisting of acetone, butanone, methyl isobutyl ketone, cyclohexanone; preferably, the ketonic solvent being present in the reaction system in amount of not more than 25 vol%; the molecular weight of the resultant polymer in the present invention can be controlled by changing the volume percentage of the organic solvent in the reaction system;

The water is present in the reaction system in amount of not more than 25 vol%; the molecular weight of the resultant polymer can be adjusted by adjusting the volume percentage of the water in the reaction system;

The initial concentration of the acrylamide monomer in the reaction system is in range of 0.1-3 mol/L; the molecular weight of the resultant polymer in the present invention can be adjusted by changing the initial concentration of the acrylamide monomer in the reaction system;

S-2, introducing the high purity nitrogen into the reactor of reaction system comprising the solvent, water and acrylamide monomer to remove oxygen, stirring, and rising the temperature of the reaction system up to the target temperature in range of 30-85°C, preferably, 40-70°C; the molecular weight of the resultant polymer can be adjusted by changing the target temperature of synthesis;

S-3, adding the initiator into the reaction system when the temperature reaches the target temperature, with stirring and introducing the nitrogen;

The initiator is an azo initiator which is one or more selected from a group consisting of 2,2-azobisisobutyronitrile, 2,2'-azobisisoheptonitrile, 2,2'-azobis[2-methylpropionamidine]dihydrochloride, diisopropyl 2,2'-azobisbutyrate, dimethyl 2,2'-azobis(2-methylpropionate);

The initiator further is a inorganic or organic peroxide, which is one or more selected from the group consisting of ammonium persulfate, sodium persulfate, potassium persulfate, tertiary butyl peroxide, dicumyl peroxide and benzoyl peroxide; a reducing agent further is added simultaneously, such as sodium bisulfite or sodium metabisulfite, to make the polymerization reaction faster and more thoroughly;

The amount of the initiator is in range of 0.01-1 wt% of the acrylamide monomer; the molecular weight of the resultant polymer in the synthetic method can be controlled by changing the amount of the initiator;

S-4, after the addition of the initiator, keeping the target temperature for a certain time with stirring and introducing the nitrogen;

Keeping the target temperature for 8-30 hours, preferably, 12-20 hours;

S-5, after the reaction, vacuum filtering a solid-liquid mixture in the reactor, then drying the resultant solid product at the temperature in range of 30-70°C;

S-6, dissolving the resultant dry product in a moderate amount of water completely, adding the organic solvent which dissolves only the acrylamide monomer not the polymerization product to re-precipitate for removing the unreacted acrylamide monomer; the organic solvent being one or more selected from the group consisting of methanol, ethanol, n-propanol, isopropanol, n-butanol, acetone, butanone, methyl isobutyl ketone and cyclohexanone;

Repeating the steps of S-5 and S-6 for 2-4 times;

S-7, drying the resultant product with the acrylamide monomer removed in a vacuum oven at the temperature in range of 30-70°C for 20-50 hours to obtain the polyacrylamides polymer; after drying, the polyacrylamides polymers is preserved in a d dry and closed container.

The resultant polyacrylamides polymer is mixed with water to obtain an aqueous solution, which is then poured into the mould to obtain the bulk material by drying.

According to the standard of GB/T4340.2, Vickers hardness of polyacrylamides polymer is about in range of 150-600 HV; according to the standard of D-256 of US ATSM, the impact strength is about in range of 5-30 J/m.

The residual amount of acrylamide monomer in polyacrylamides polymer is measured by SHIMADZU liquid chromatograph (LC-20A/SPD-20AV), the measured values of amount of acrylamide monomer accounting for the total amount of the polyacrylamide are not more than 0.5 ppm.

According to GB 17514-2008 method and static light scattering method (Wyatt DAWN HELEOS-II), the molecular weight of polymer prepared in step S-7 is measured; the measured value of molecular weight of polymer under different synthesis conditions is in range of 1.0×10⁴-2.0×10⁵.

To solve the above second technical problem, the present invention provides a method for preparing the polymer micro-needle array chip, comprising the following steps:
1) a prototype of micro-needle array chip with the same spatial features of the target polymer micro-needle array chip is prepared using a metal material;
2) a cavity die is prepared using the prototype of metal micro-needle array chip prepared in step 1) and a polymer material;
3) removing the prototype of micro-needle array chip from the cavity die;
4) mixing the polyacrylamides polymer with water to obtain an aqueous solution;
5) pouring the aqueous solution prepared in step 4) into the cavity die;
6) drying the aqueous solution poured into the cavity die to cure the polyacrylamides polymer of the aqueous solution in the cavity die and obtain the polymer micro-needle array chip.

Furthermore, the metal material in step 1) is one or more selected from the group consisting of titanium, copper, aluminum, nickel, tungsten, stainless steel, titanium alloy, nickel alloy, aluminum alloy and copper alloy.

Furthermore, the polymer material in step 2) is one or more selected from the group consisting of polyethylene, polypropylene, polylactic acid, poly (butylene succinate) and polydimethylsiloxane.

Furthermore, in step 4), mixing the polyacrylamides polymer with water to obtain an aqueous solution at the temperature in range of 10-90°C; the polyacrylamides polymer being present in the aqueous solution in amount of 1-80 mass%, preferably, 10-50 mass%; preferably, ultrasonic processing the aqueous solution to remove bubbles;

Furthermore, in step 5), pouring the aqueous solution prepared in step 4) into the cavity die cleaned with water, and placing the cavity die with the aqueous solution onto a horizontal operation platform; preferably, placing the cavity die and the horizontal operation platform in a closed system; preferably, sealing the horizontal operation platform and the cavity die with a viscous liquid;

Furthermore, in step 6), the drying temperature is in range of 20-90°C.

Furthermore, step 5) and step 6) comprise the following specific steps:

F1, in case that the polyacrylamides polymer of the aqueous solution for a once-through pour in the cavity die is sufficient to prepare the polymer micro-needle array chip, drying the aqueous solution in the cavity die directly and curing the polyacrylamides polymer therein to obtain the polymer micro-needle array chip;

G1, in case that the polyacrylamides polymer of the aqueous solution for a once-through pour in the cavity die is not sufficient to prepare the polymer micro-needle array chip, drying the aqueous solution in the cavity die to remove part of the water therein; pouring for a second time or more times until the polyacrylamides polymer in the cavity die is sufficient to prepare the polymer micro-needle array chip; drying the aqueous solution and curing the polyacrylamides polymer to obtain the polymer micro-needle array chip.

The present invention provides a method for preparing the polymer micro-needle array chip, comprising the following steps:
1) a prototype of micro-needle array chip with the same spatial features of the target polymer micro-needle array chip is prepared using a metal material;
2) a cavity die is prepared using the prototype of metal micro-needle array chip prepared in step 1) and a polymer material;
3) removing the prototype of micro-needle array chip from the cavity die;
4) mixing the polyacrylamides polymer with the bioactive substances or drugs to obtain a mixture;
5) mixing the mixture prepared in step 4) with water to obtain the mixture solution;
6) pouring the mixture solution prepared in step 5) into the cavity die;
7) drying the mixture solution to obtain the polymer micro-needle array chip.

Furthermore, the metal material in step 1) is one or more selected from the group consisting of titanium, copper, aluminum, nickel, tungsten, stainless steel, titanium alloy, nickel alloy, aluminum alloy and copper alloy.

Furthermore, the polymer material in step 2) is one or more selected from the group consisting of polyethylene, polypropylene, polylactic acid, poly (butylene succinate) and polydimethylsiloxane.

Furthermore, in the mixture obtained in step 4) comprising the polyacrylamides polymer and the bioactive substances or drugs, the bioactive substances or drugs is present in the mixture in amount of 0.1-50 mass%, preferably, 10-20 mass%;

Furthermore, in step 5), mixing the resultant mixture in step 4) with water to obtain an mixture solution at the temperature in range of 10-90°C; the mixture being present in the mixture solution in amount of 1-80 mass%, preferably, 10-50 mass%; preferably, ultrasonic processing the mixture solution to remove bubbles;

Furthermore, in step 6), pouring the mixture solution prepared in step 5) into the cavity die cleaned with water, and placing the cavity die with the aqueous solution onto a horizontal operation platform; preferably, placing the cavity die and the horizontal operation platform in a closed system; preferably, sealing the horizontal operation platform and the cavity die with a viscous liquid;

Furthermore, in step 7), the drying temperature is in range of 20-90°C.

Furthermore, step 6) and step 7) comprise the following specific steps:

H2, in case that the mixture of the mixture solution for a once-through pour in the cavity die is sufficient to prepare the polymer micro-needle array chip, drying the mixture solution in the cavity die directly and curing the mixture to obtain the polymer micro-needle array chip;

I2, in case that the mixture of the mixture solution for a once-through pour in the cavity die is not sufficient to prepare the polymer micro-needle array chip, drying the mixture solution in the cavity die to remove part of the water therein; pouring for a second time or more times until the mixture in the cavity die is sufficient to prepare the polymer micro-needle array chip; drying the mixture solution and curing the mixture to obtain the polymer micro-needle array chip.

The present invention provides a method for preparing the polymer micro-needle array chip, comprising the following steps:
1) a prototype of micro-needle array chip with the same spatial features of the target polymer micro-needle array chip is prepared using a metal material;
2) a cavity die is prepared using the prototype of metal micro-needle array chip prepared in step 1) and a polymer material;
3) removing the prototype of micro-needle array chip from the cavity die;
4) mixing the polyacrylamide polymer with the bioactive substances or drugs to obtain a mixture;
5) mixing the resultant mixture in step 4) with water to obtain the mixture solution;
6) pouring the resultant mixture solution in step 5) into the cavity die;
7) drying the mixture solution poured into the cavity die to cure the mixture in the cavity die and obtain the micro-needle array and the substrate film connected thereto, of the polymer micro-needle array chip;
8) mixing the polyacrylamides polymer with water to obtain the aqueous solution;
9) pouring the aqueous solution prepared in step 8) continually into the cavity die in step 8);
10) drying the aqueous solution in step 9) poured into the cavity die, curing the polyacrylamides polymer of the aqueous solution poured into the cavity die to obtain entire polymer micro-needle array chip.

Furthermore, the metal material in step 1) is one or more selected from the group consisting of titanium, copper, aluminum, nickel, tungsten, stainless steel, titanium alloy, nickel alloy, aluminum alloy and copper alloy.

Furthermore, the polymer material in step 2) is one or more selected from the group consisting of polyethylene, polypropylene, polylactic acid, poly(butylene succinate) and polydimethylsiloxane.

Furthermore, in step 4), in the mixture comprising the polyacrylamides polymer and the bioactive substances or drugs, the bioactive substances or drugs is present in the mixture in amount of 0.1-50 mass%, preferably, 10-20 mass%;

Furthermore, in step 5), mixing the resultant mixture in step 4) with water to obtain an mixture solution at the temperature in range of 10-90°C; the mixture being present in the mixture solution in amount of 1-80 mass%, preferably, 10-50 mass%; preferably, ultrasonic processing the mixture solution to remove bubbles;

Furthermore, in step 6), pouring the mixture solution prepared in step 5) into the cavity die cleaned with water, and placing the cavity die with the aqueous solution onto a horizontal operation platform; preferably, placing the cavity die and the horizontal operation platform in a closed system; preferably, sealing the horizontal operation platform and the cavity die with a viscous liquid;

Furthermore, in step 7), the drying temperature is in range of 20-90°C.

Furthermore, step 6) and step 7) comprise the following specific steps:

H3, in case that the aqueous solution or the mixture of the mixture solution for a once-through pour in the cavity die is sufficient to prepare the polymer micro-needle array and the substrate film of the polymer micro-needle array chip, drying the aqueous solution or the mixture solution in the cavity die directly and curing the mixture to obtain the polymer micro-needle array and the substrate film of the polymer micro-needle array chip;

I3, in case that the aqueous solution or the mixture of the mixture solution for a once-through pour in the cavity die is not sufficient to prepare the polymer micro-needle array and the substrate film of the polymer micro-needle array chip, drying the aqueous solution or the mixture solution in the cavity die to remove part of the water therein; pouring for a second time or more times until the mixture in the cavity die is sufficient to prepare the polymer micro-needle array and the substrate film of the polymer micro-needle array chip; drying the mixture solution and curing the mixture to obtain the polymer micro-needle array and the substrate film of the polymer micro-needle array chip, as designed.

Furthermore, in step 8), mixing the polyacrylamides polymer with water to obtain an aqueous solution at the temperature in range of 10-90°C; the polyacrylamide polymer being present in the aqueous solution in amount of 20-80 mass%, preferably, 40-60 mass%; preferably, ultrasonic processing the mixture solution to remove bubbles.

Furthermore, in step 9), the concentration of the polyacrylamide polymer in aqueous solution is higher than that in the mixture solution prepared in step 5); this is for reducing the diffusion of the bioactive substances or drugs contained in the micro-needle array prepared in the above step 1)-step7) into the substrate.

Furthermore, in step 10), the drying temperature is in range of 20-90°C.

The present invention provides a method for preparing the polymer micro-needle array chip, comprising the following steps:
1) a prototype of micro-needle array chip with the same spatial features of the target polymer micro-needle array chip is prepared using a metal material;
2) a cavity die is prepared using the prototype of metal micro-needle array chip prepared in step 1) and a polymer material;
3) removing the prototype of micro-needle array chip from the cavity die;
4) mixing the polyacrylamide polymer with water to obtain an aqueous solution;
5) pouring the aqueous solution prepared in step 4) into the cavity die;
6) drying the aqueous solution poured into the cavity die to cure the polyacrylamides polymer in the cavity die and obtain the micro-needle array and the substrate film of the polymer micro-needle array chip;
7) connecting the micro-needle array and the substrate film of the polymer micro-needle array chip prepared in step 6) with one or more film material to obtain the polymer micro-needle array chip;

Furthermore, the metal material in step 1) is one or more selected from the group consisting of titanium, copper, aluminum, nickel, tungsten, stainless steel, titanium alloy, nickel alloy, aluminum alloy and copper alloy.

Furthermore, the polymer material in step 2) is one or more selected from the group consisting of polyethylene, polypropylene, polylactic acid, poly(butylene succinate) and polydimethylsiloxane.

Furthermore, in step 4), mixing the polyacrylamides polymer with water to obtain an aqueous solution at the temperature in range of 10-90°C; the polyacrylamide polymer being present in the aqueous solution in amount of 1-80 mass%, preferably, 10-50 mass%; preferably, ultrasonic processing the mixture solution to remove bubbles.

Furthermore, in step 5), pouring the aqueous solution prepared in step 4) into the cavity die cleaned with water, and placing the cavity die onto a horizontal operation platform; preferably, placing the cavity die and the horizontal operation platform in a closed system; preferably, sealing the horizontal operation platform and the cavity die with a viscous liquid.

Furthermore, in step 6), the drying temperature is in range of 20-90°C.

Furthermore, step 6) comprises the following specific steps:

F4, in case that the polyacrylamides polymer in aqueous solution for a once-through pour in the cavity die is sufficient to prepare the micro-needle array and the substrate film of the polymer micro-needle array chip, drying the aqueous solution in the cavity die directly and curing the polyacrylamides polymer to obtain the micro-needle array and the substrate film of the polymer micro-needle array chip, as designed;

G4, in case that the polyacrylamides polymer in aqueous solution for a once-through pour in the cavity die is not sufficient to prepare the micro-needle array and the substrate film of the polymer micro-needle array chip, drying the aqueous solution in the cavity die to remove part of the water therein; pouring for a second time or more times until the polyacrylamides polymer in the cavity die is sufficient to prepare the micro-needle array and the substrate film of the polymer micro-needle array chip; drying the aqueous solution and curing the polyacrylamides polymer to obtain the micro-needle array and the substrate film of the polymer micro-needle array chip, as designed.

Furthermore, in step 7), the one or more film is a combination of one or more selected from the group consisting of polyethylene, polypropylene, poly(butylene succinate), polydimethylsiloxane, rubber, polylactic acid, latex, glass and metal thermoplastic composite materials; the one or more film is combined tightly with the substrate film by cohering, fusion and bonding.

The present invention provides a method for preparing the polymer micro-needle array chip, comprising the following steps:
1) a prototype of micro-needle array chip with the same spatial features of the target polymer micro-needle array chip is prepared using a metal material;
2) a cavity die is prepared using the prototype of metal micro-needle array chip prepared in step 1) and a polymer material;
3) removing the prototype of micro-needle array chip from the cavity die;
4) mixing the polyacrylamides polymer with the bioactive substances or drugs to obtain a mixture;
5) mixing the mixture prepared in step 4) with water to obtain the mixture solution;
6) pouring the mixture solution prepared in step 5) into the cavity die;
7) drying the aqueous solution or the mixture solution of the mixture composed of the polyacrylamide polymer and the bioactive substances or drugs poured into the cavity die to cure the mixture and obtain the micro-needle array and the substrate film of the polymer micro-needle array chip;
8) connecting the micro-needle array and the substrate film with the thickness of less than 50 µm of the polymer micro-needle array chip prepared in step 7), with one or more film material to obtain the polymer micro-needle array chip.

Furthermore, the metal material in step 1) is one or more selected from the group consisting of titanium, copper, aluminum, nickel, tungsten, stainless steel, titanium alloy, nickel alloy, aluminum alloy and copper alloy.

Furthermore, the polymer material in step 2) is one or more selected from the group consisting of polyethylene, polypropylene, polylactic acid, poly (butylene succinate) and polydimethylsiloxane.

Furthermore, in step 4), in the mixture comprising the polyacrylamides polymer and the bioactive substances or drugs, the bioactive substances or drugs is present in the mixture in amount of 0.1-50 mass%, preferably, 10-20 mass%;

Furthermore, in step 5), mixing the mixture with water to obtain an mixture solution at the temperature in range of 10-90°C; the mixture being present in the mixture solution in amount of 1-80 mass%, preferably, 10-50 mass%; preferably, ultrasonic processing the mixture solution to remove bubbles;

Furthermore, in step 6), pouring the mixture solution prepared in step 5) into the cavity die cleaned with water, and placing the cavity die with the aqueous solution onto a horizontal operation platform; preferably, placing the cavity die and the horizontal operation platform in a closed system; preferably, sealing the horizontal operation platform and the cavity die with a viscous liquid;

Furthermore, in step 7), the drying temperature is in range of 20-90°C.

Furthermore, step 6) and step 7) comprise the following specific steps:

H5, in case that the mixture of the mixture solution for a once-through pour in the cavity die is sufficient to prepare the polymer micro-needle array and the substrate film of the polymer micro-needle array chip, drying the aqueous solution or the mixture solution in the cavity die directly and curing the mixture to obtain the polymer micro-needle array and the substrate film of the polymer micro-needle array chip, as designed;

I5, in case that the mixture of the mixture solution for a once-through pour in the cavity die is not sufficient to prepare the polymer micro-needle array and the substrate film of the polymer micro-needle array chip, drying the aqueous solution or the mixture solution in the cavity die to remove part of the water therein; pouring for a second time or more times until the mixture in the cavity die is sufficient to prepare the polymer micro-needle array and the substrate film of the polymer micro-needle array chip; drying the mixture solution and curing the mixture to obtain the polymer micro-needle array and the substrate film of the polymer micro-needle array chip, as designed.

Furthermore, in step 8), the one or more film is one or more selected from the group consisting of polyethylene, polypropylene, poly(butylene succinate), polydimethylsiloxane, rubber, polylactic acid, latex, glass and metal thermoplastic composite materials; preferably, the one or more film is combined tightly with the substrate film by cohering, fusion and bonding.

To solve the above first technical problem, a technical solution provided by the present invention is as follows:
a patch for transdermal drug delivery prepared through utilizing the polymer micro-needle array chip comprises a polymer micro-needle array chip, a base plate, an anti-seepage washer, an anti-adhesion layer, an adhesive tape and an anti-seepage layer; further comprises a micro-needle array protective device;
the polymer micro-needle array chip comprises a substrate and a micro-needle array standing thereon, which is the core component of the patch for micro-needle transdermal drug delivery;
the micro-needle array protective device is a device to protect the micro-needle array not damaged by the external environment before it's used; preferably, the mould used for preparing the polymer micro-needle array chip acts as the micro-needle array protective device;
the base plate is one or more film which is adhesive to the back of the substrate of the polymer micro-needle array chip; so that the stratum corneum of the skin can be easily pierced by micro-needle when the medicine is used.
the anti-seepage washer is a layer of washer with the protective effect surround the edge of the substrate of the polymer micro-needle array chip and the base plate of the micro-needle patch.
the anti-adhesion layer is a film which covers the area outside of the micro-needle chip, and the anti-adhesion layer that is easy to peel off when the medicine is used;
the adhesive tape is a double sided sticky tape which is sticky on both sides, one side of the tape covers the base plate, the anti-seepage washer and the anti-adhesion layer, the other side of the tape is adhered to the anti-seepage layer; the adhesive tape has mainly the effect of fixing when the medicine is used;
the anti-seepage layer is a layer of protective film covering the outside of the adhesive tape, mainly in order to prevent the micro-needle array chip inside the patch affected by the external environment.

A method for preparing a patch for transdermal drug delivery through utilizing the polymer micro-needle array chip of the present invention mainly comprises the following steps:
1. adhering the base plate to the substrate of the polymer micro-needle array chip;
2. fixing the anti-seepage washer around the base plate of the micro-needle patch;
3. placing the anti-adhesion layer at the outside of the micro-needle base plate or that of the substrate of the polymer micro-needle array chip;
4. adhering one side of the double sided sticky tape to the base plate, the anti-seepage washer and the anti-adhesion layer of the micro-needle patch;
5. adhering the anti-seepage layer to the other side of the adhesive tape so as to obtain the patch for polymer transdermal drug delivery.

The present invention has the following advantages:
1. the micro-needle of the polymer micro-needle array chip of the present invention has a high mechanical strength and a sharp needle tip, so the stratum corneum of the skin can be easily pierced;
2. The matrix material of the micro-needle array of the polymer micro-needle array chip of the present invention is a water soluble polyacrylamides polymer, a polymer micro-needle array chip can be prepared through the way of moulding, using the solution of the polyacrylamides polymer, the aqueous solution or the mixture solution composed of the polyacrylamides polymer and the bioactive substances or drugs; the preparation method avoids a high-temperature processing step, and is in favor of maintaining the activities of biomacromolecules drugs comprising polypeptides, proteins and the like;
3. The polyacrylamides polymer of the present invention can easily dissolve or swell on contact with a water-containing environment, which helps the drug to be released slowly in the skin.
4. The method for preparing the patch for transdermal drug delivery based on the polymer micro-needle array chip is simple, so that mass production can be realized by current processing technologies.

### DESCRIPTION OF THE DRAWINGS

Fig. 1 is a schematic diagram of a micro-needle array chip;
Fig. 2-1 is a schematic diagram showing the structure of the needle body of a micro-needle;
Fig. 2-2 is a schematic diagram showing the structure of the needle body of a micro-needle;

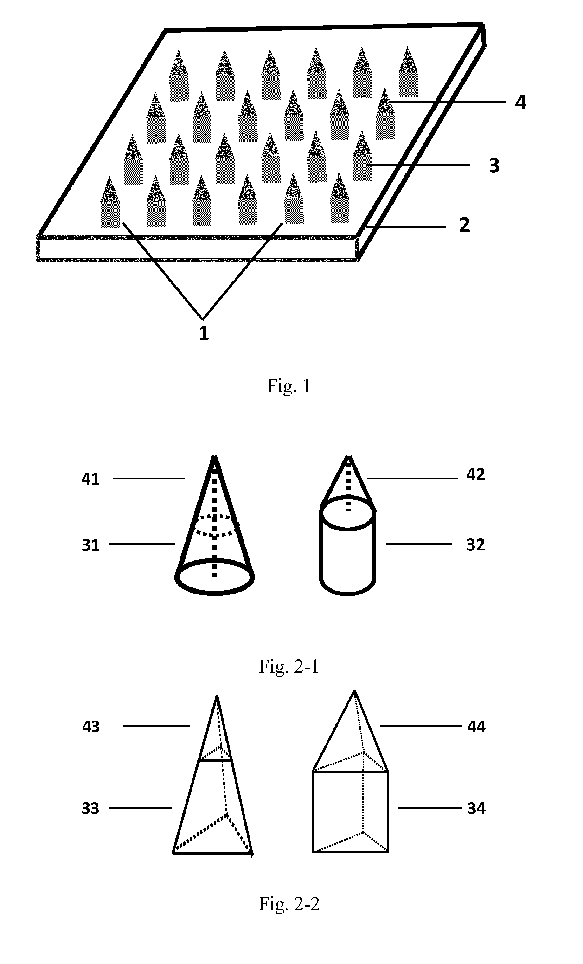

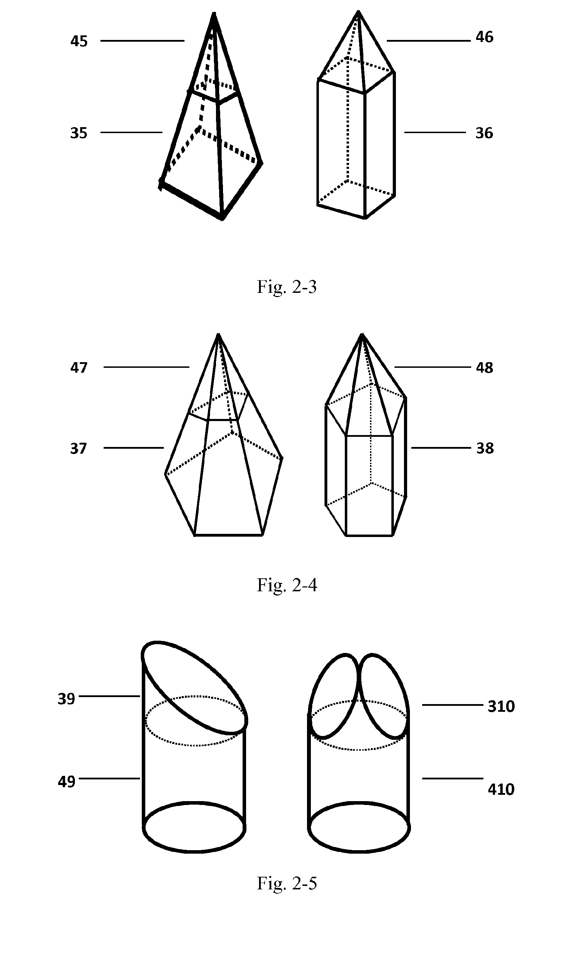

Fig. 2-3 is a schematic diagram showing the structure of the needle body of a micro-needle;
Fig. 2-4 is a schematic diagram showing the structure of the needle body of a micro-needle;
Fig. 2-5 is a schematic diagram showing the structure of the needle body of a micro-needle;

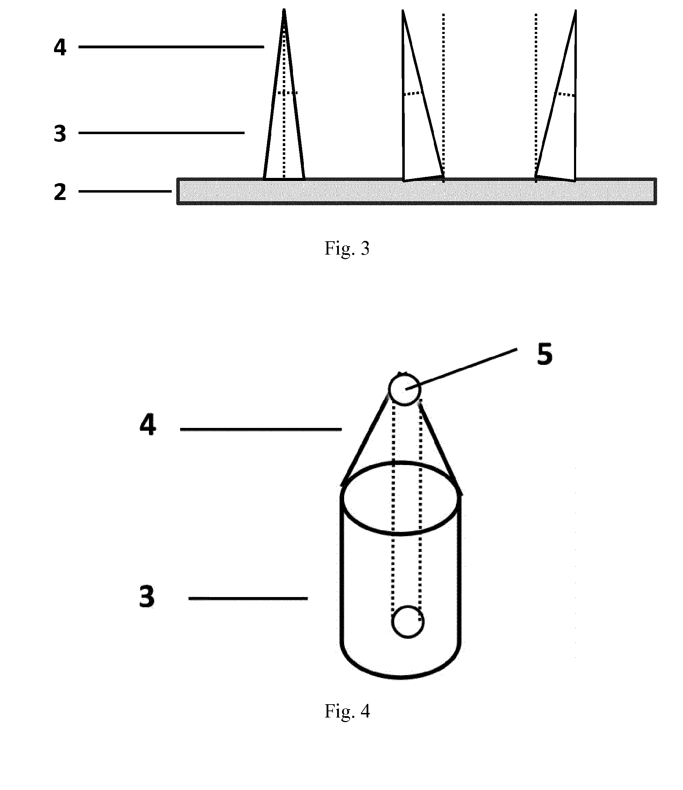

Fig. 3 is a sectional view showing the structure of a micro-needle array chip, of which the axis of micro-needles is vertical to or inclined toward the plane of the substrate;
Fig. 4 is a schematic diagram of a hollow micro-needle;

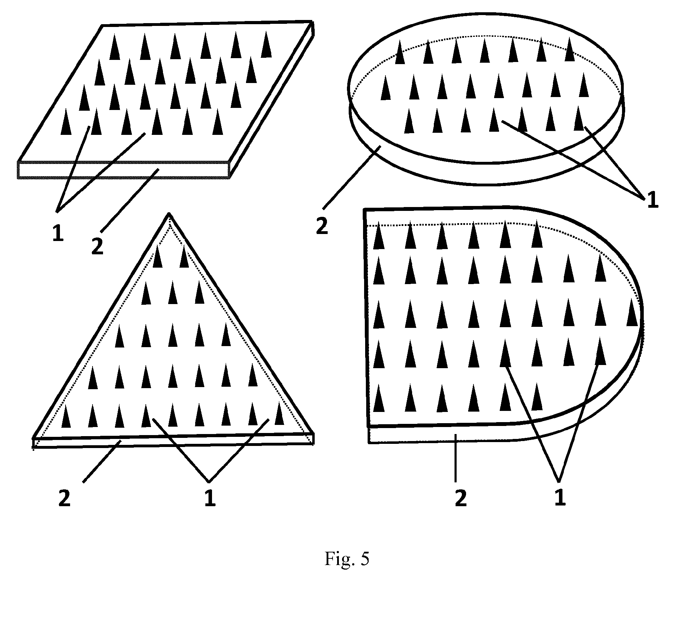

Fig.5 is a schematic diagram of a micro-needle array chip, of which the micro-needle array is rectangular, oval, triangular and irregular shape;

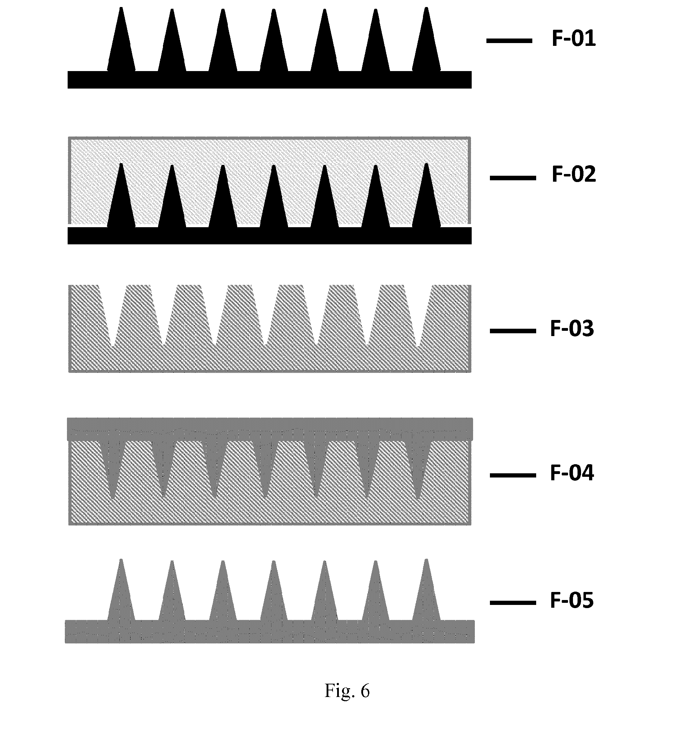

Fig.6 is a schematic diagram of a technical route for preparing a polymer micro-needle array chip;

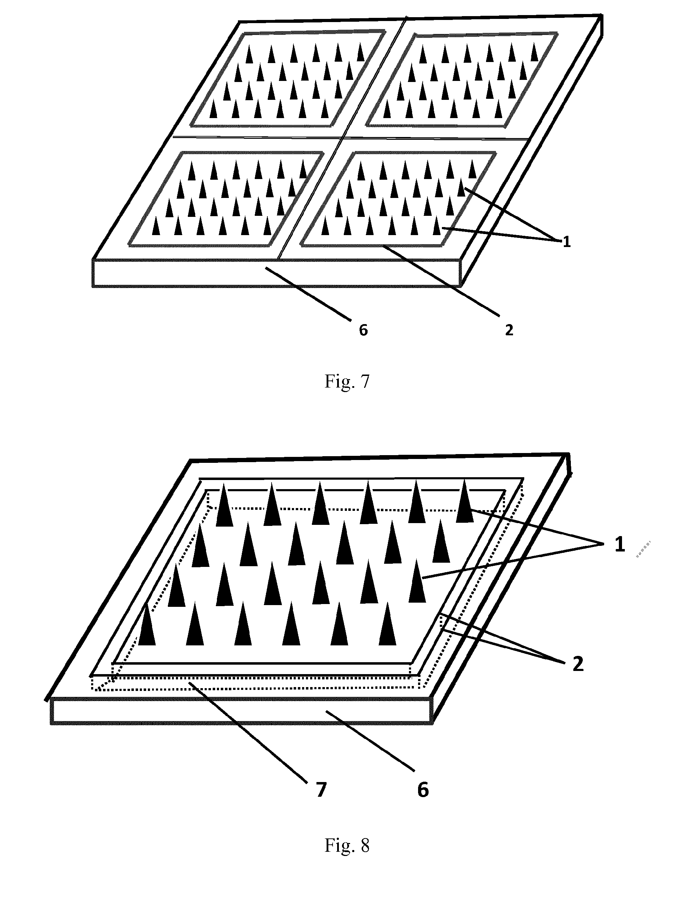

Fig.7 is a schematic diagram of a micro-needle array chip, when multiple prototypes of type A micro-needle rank on the same smooth flat base surface 6;
Fig.8 is a schematic diagram of the prototype of type B micro-needle array;

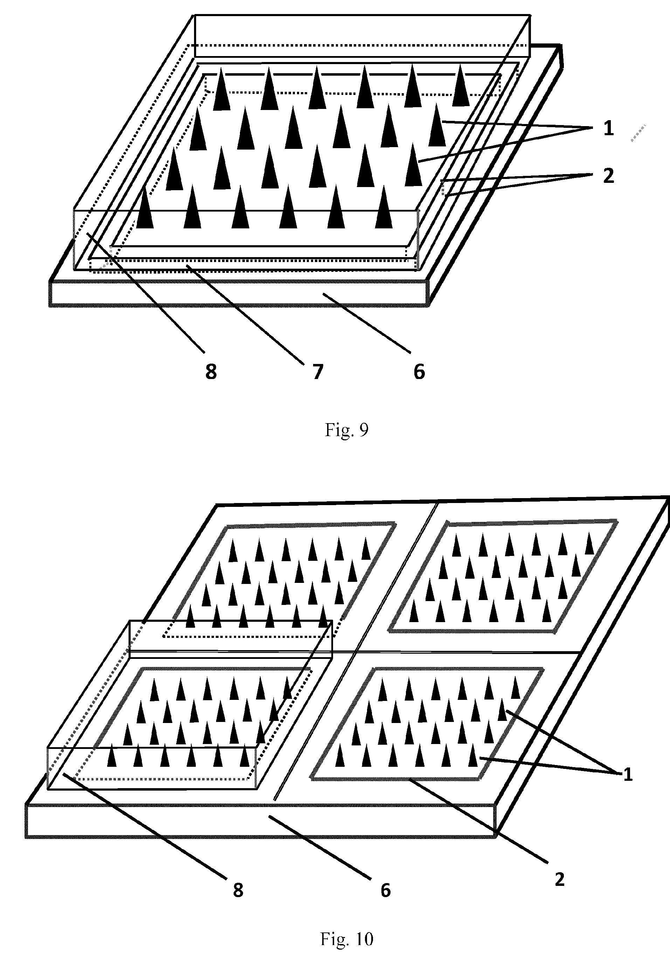

Fig.9 is a schematic diagram of the prototype of type C micro-needle array;
Fig.10 is a schematic diagram showing the three-dimensional structure of the cavity die prepared by using the prototype of type A micro-needle array chip;

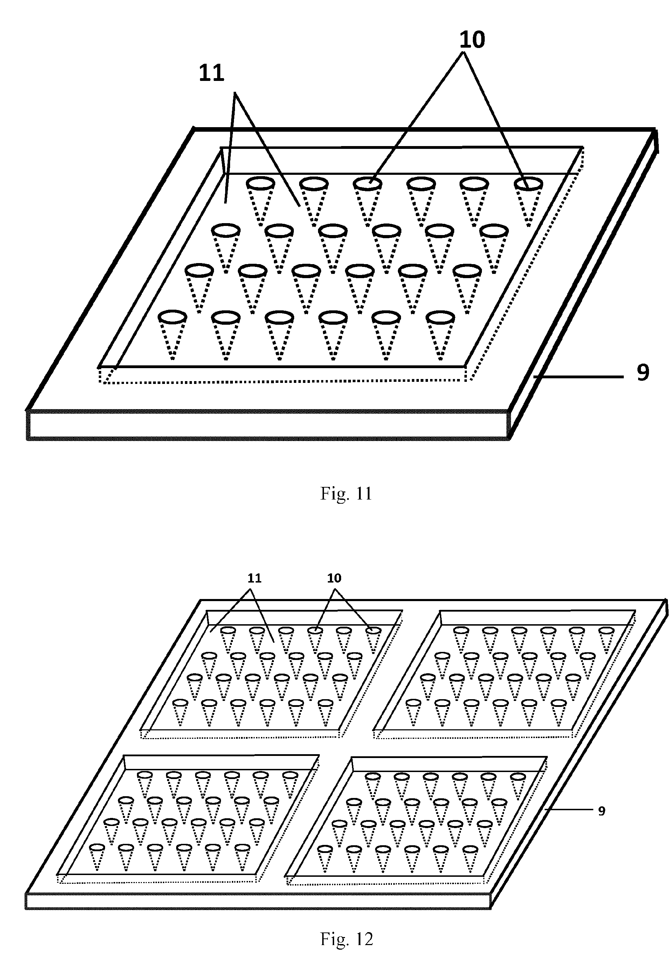

Fig.11 is a schematic diagram of single cavity die used for preparing a micro-needle array chip;
Fig. 12 is a schematic diagram of the template containing multiple cavity die;

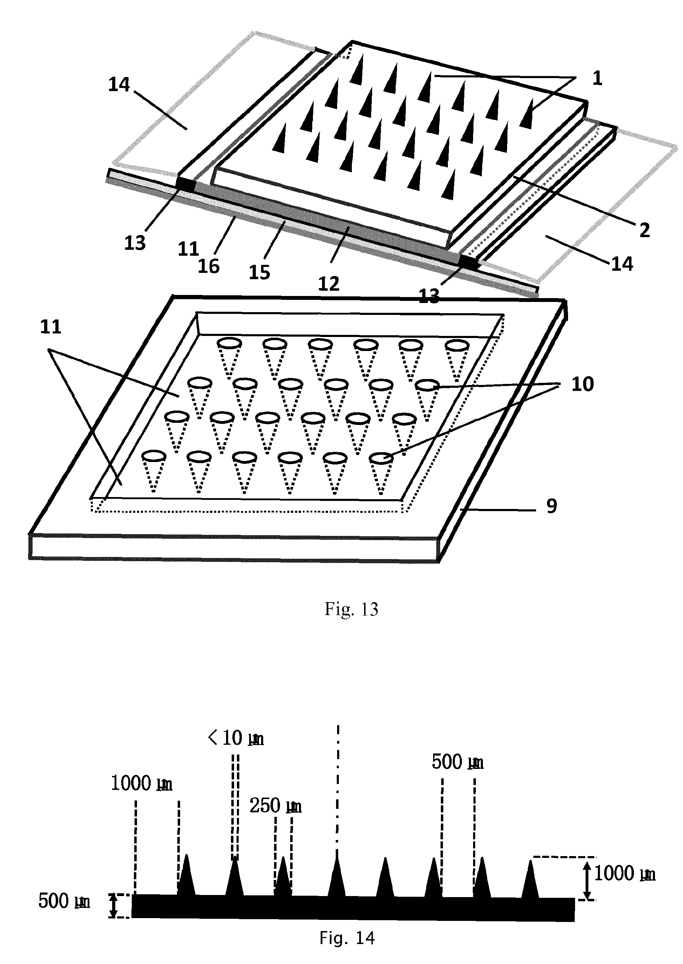

Fig.13 is a schematic diagram of the preparation of micro-needle transdermal drug delivery;
Fig.14 is a cross-sectional schematic of the prototype of type A micro-needle array chip;

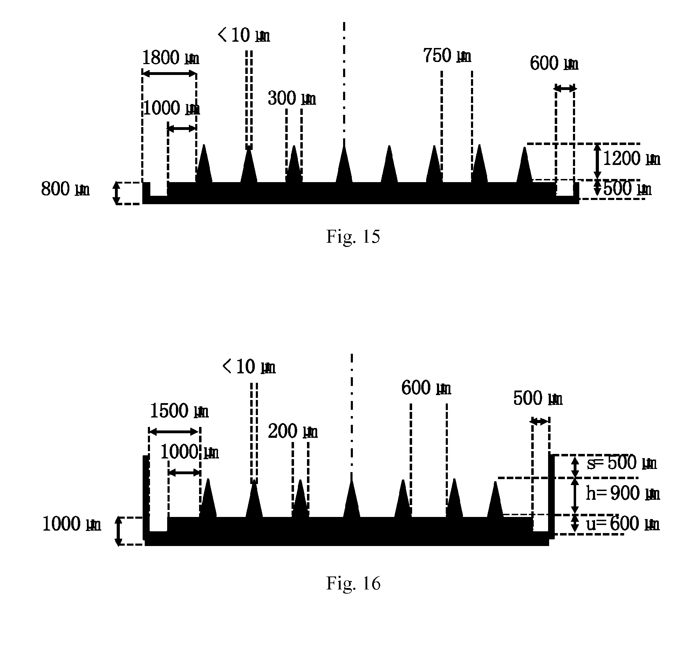

Fig. 15 is a cross-sectional schematic of the prototype of type B micro-needle array chip;
Fig.16 is a cross-sectional schematic of the prototype of type C micro-needle array chip;

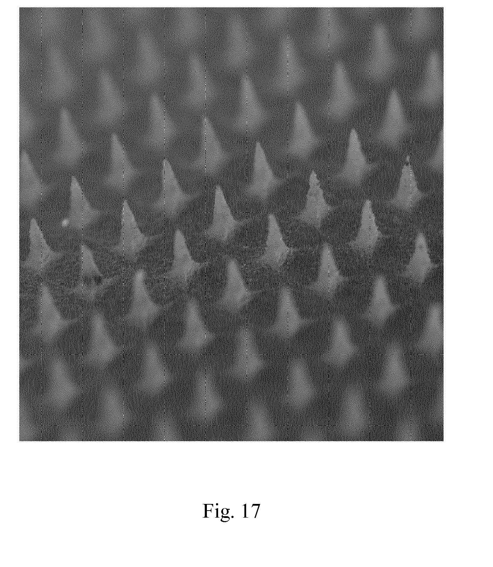

Fig. 17 is an overall topography of a solid polymer micro-needle array chip prepared in an example of the invention.

### DETAILED DESCRIPTION OF THE INVENTION

The following embodiments is further details of the present invention for the polymer micro-needle array chip, and preparation and application thereof. The aim is to enable those skilled in the art to have better understanding for the present invention.

The micro-needle array chip of the present invention as shown in Fig.1, comprises a substrate 2 and a micro-needle array 1 standing thereon, and the matrix material of the micro-needle array 1 is a polyacrylamides polymer;

The polyacrylamides polymer polymerized from acrylamide monomer is a medical polymer, and the reaction equation is as follows:

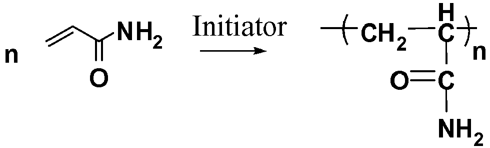

The structure of the polymer is straight-chain homopolymer, copolymer or crosslinked polymer. Preferably, the polymer is straight-chain homopolymer, wherein the molecular weight is 1.0×10⁴-2.0×10⁵; the Vickers hardness is 150-600 HV; the impact strength is 5-30 J/M; the amount of residual acrylamide monomer is not more than 0.5 ppm, which meets the medical standards prescribed by the World Health Organization.

The polyacrylamides polymer has a good solubility in water, which can be mixed with water to obtain an aqueous solution with the polymer in amount of 1-80 mass%. A square sheet prepared by the polyacrylamides polymer and having a thickness of 2 mm and sides of length 1 cm, dissolves at least 50 vol% after immersing in a still physiological saline for about 6 hours.

The micro-needle array 1 of the polymer micro-needle array chip is made of the polyacrylamides polymer, or a mixture composed of the polyacrylamide polymer and bioactive substances or drugs.

The substrate 2 of the polymer micro-needle array chip is made of the pure polyacrylamide polymer, or a mixture composed of the polyacrylamides polymer and bioactive substances or drugs, a combination of one or more layers selected from the group consisting of polylactic acid, polyethylene, polypropylene, poly(butylene succinate), rubber, latex, glass and metal thermoplastic composite materials.

The bioactive substances or drugs is one or more selected from vaccines, polypeptides, proteins, polysaccharides, nucleic acids, hormones, anti-cancer drugs, genetic engineering drugs, natural product drugs, the traditional Chinese medicine or nutrients with any molecular weight; the bioactive substances or drugs being present in the mixture composed of the polyacrylamide polymer and the bioactive substances or drugs in amount of 0.1-50 mass%, preferably, 10-20 mass%; the mixing ratio can be adjusted according to the dose required of the bioactive substances or drugs and the spatial feature of the polymer micro-needle array chip; the mixture prepared is mixed with water to obtain an aqueous solution or a mixture solution with fluidity; the mixture is present in an aqueous solution or a mixture solution in amount of 1-80 mass%, preferably, 10-50 mass%.

The present invention provides a synthetic method for preparing the polyacrylamides polymer, mainly comprising the following steps:

S-1, according to the set value, an organic solvent, water and acrylamide monomer are added to a reactor equipped with a stirring device;

The organic solvent is mainly alcohols, secondly is ketones. The alcohols are at least one selected from the group consisting of methanol, ethanol, n-propanol, isopropanol, n-butanol and other components, or a mixture of two or more of them. Preferably, the alcohols solvent is present in the reaction system not less than 60 vol%; the ketones is one or more selected from the group consisting of acetone, butanone, methyl isobutyl ketone, cyclohexanone and other components; preferably, the ketones solvent is present in the reaction system not more than 25 vol%. The molecular weight of the polymer prepared in the present invention can be controlled by changing the volume percentage of the organic solvent in the reaction system;

The water is present in the reaction system not more than 25 vol%; the molecular weight of the polymer prepared can be adjusted by adjusting the volume percentage of the water in the reaction system.

The initial concentration of the acrylamide monomer in the reaction system is 0.1-3 mol/L; the molecular weight of the polymer prepared in the present invention can be adjusted by changing the initial concentration of the acrylamide monomer in the reaction system;

S-2, introducing the high purity nitrogen into the reactor of reaction system composed of the solvent and acrylamide monomer to remove oxygen, with stirring, and the reaction system is heated up to a target temperature simultaneously; the target temperature is in range of 30-85°C, preferably, 40-70°C; the present invention can adjust the molecular weight of the polymer prepared by changing the target temperature of synthesis;

S-3, when the temperature of the reaction system reaches the target temperature, adding the initiator to the above reaction system with stirring and introducing nitrogen;

The initiator can be an azo initiator, such as is at least one selected from 2,2-azobisisobutyronitrile, 2,2'-azobisisoheptonitrile, 2,2'-azobis[2-methylpropionamidine]dihydrochloride, diisopropyl 2,2'-azobisbutyrate, dimethyl 2,2'-azobis(2-methylpropionate), or a mixture of two or more of them;

The initiator can also be inorganic or organic peroxide, such as is at least one selected from the group consisting of ammonium persulfate, sodium persulfate, potassium persulfate, tertiary butyl peroxide, dicumyl peroxide and benzoyl peroxide; a reducing agent can also be added simultaneously, such as sodium bisulfite or sodium metabisulfite, to make the polymerization reaction faster and more thoroughly;

The amount of the initiator is generally in range of 0.01-1wt% of the above acrylamide monomer; the molecular weight of the polymer prepared in the synthetic method can be controlled by changing the amount of the initiator;

S-4, after adding the initiator, keeping the target temperature for a certain time, with stirring and introducing nitrogen.

The duration for keeping the reaction system at the target temperature in the present invention is 8-30 hours, preferably, 12-20 hours;

S-5, after the reaction, vacuum filtering the solid-liquid mixture in the reactor first, and then drying the resultant solid product in a vacuum oven at the temperature of 30-70°C;

S-6, dissolving the resultant dry product in moderate water completely, adding the organic solvent which dissolves only the acrylamide monomer not the polymerization product to re-precipitate for removing the unreacted acrylamide monomer; the organic solvent being one or more selected from the group consisting of methanol, ethanol, n-propanol, isopropanol, n-butanol, acetone, butanone, methyl isobutyl ketone and cyclohexanone;

Repeating the steps of S-5 and S-6 for three times;

S-7, drying the resultant product with the acrylamide monomer removed in a vacuum oven at the temperature in range of 30-70°C for 20-50 hours, preserving the resultsant samples of polyacrylamides polymer in a dry and closed container.

S-8, the residual amount of acrylamide monomer in the samples prepared in step S-7 is measured by SHIMADZU liquid chromatograph (LC-20A/SPD-20AV), and the measured values of amount of acrylamide monomer accounting for the total amount of the polyacrylamide are not more than 0.5 ppm.

S-9, according to GB 17514-2008 method and static light scattering method (Wyatt DAWN HELEOS-II), the molecular weight of polymer prepared in step S-7 is measured; the measured values of molecular weight of polymer under different synthesis conditions are 1.0×10⁴-2.0×10⁵.

S-10, mixing the polymer prepared with water to obtain an aqueous solution, and then pouring the aqueous solution into the mould to obtain the bulk material by drying; according to the standard of GB/T4340.2, Vickers hardness of the bulk material is about 150-600 HV; according to the standard of D-256 of US ATSM, the impact strength is about 5-30J/m.

The micro-needle array 1 of the polymer micro-needle array chip comprises at least two micro-needles; the micro-needle comprises a needle bar 3 and a needle head 4; the needle bar 3 is the main body of the micro-needle, one end of which is connected with the substrate 2 of the micro-needle array chip; the needle head 4 is the tip section which is at the top of the micro-needle.

The structure of micro-needle is any type of many types as shown in Fig. 2-1 to Fig. 2-5:

The needle bar 31 and the needle head 41 is a integrated conical structure; the diameter of cross-section circular in the junction between the needle bar 31 and the substrate 2 is in range of 20-3000 µm; the sum of the height of the needle bar 31 and the needle head 41 is in range of 50-5000 µm; preferably, the diameter of the cross-section circular is in range of 50-1000 µm, and the sum of the height of the needle bar 31 and the needle head 41 is in range of 200-2000 µm; the radius of curvature of the circumcircle in the tip of the needle head 41 is in range of 50 nm-300 µm; preferably, less than 10 µm.

The needle bar 32 is a cylinder, and the needle head 42 is a cone; the diameter of cross-section circular of the needle bar 32 is in range of 20-3000 µm; the height of the needle bar 32 is in range of 50-3000 µm; preferably, the diameter of the cross-section circular is in range of 50-1000 µm, the height of the needle bar 32 is in range of 200-2000 µm; the diameter of the bottom of the needle 42 is in line with the diameter of cross-section circular of the needle bar 32, and the height of the needle 42 is in range of 50-2000 µm; preferably, 50-1000 µm; the radius of curvature of the circumcircle in the tip of the needle head 42 is in range of 50 nm-300 µm; preferably, less than 10 µm.

The needle bar 33 and the needle head 43 is a integrated triangular pyramid structure; the needle bar 35 and the needle head 45 is a integrated rectangular pyramid structure; the needle bar 37 and the needle head 47 is a integrated pentagonal pyramid structure; the diameter of circumcircle circle in the junction between pyramid and the substrate 2 is in range of 20-3000 µm; the sum of the height of the needle bar 3 and the needle head 4 is in range of 50-3000 µm; preferably, the diameter of circumcircle circle is in range of 50-1000 µm; the sum of the height of the needle bar 3 and the needle head 4 is in range of 200-2000 µm; the radius of curvature of the circumcircle in the tip of the pyramidal needle head 4 is in range of 50 nm-300 µm; preferably, less than 10 µm.

The needle bar 34 is triangular prism and the needle head 44 is triangular pyramid; the needle bar 36 is quadruple prism and the needle head 46 is rectangular pyramid; the needle bar 38 is pentagonal prism and the needle head 48 is pentagonal pyramid; the diameter of cross-section circular of the prismatic needle bar 3 is in range of 20-3000 µm and the height of the needle bar 3 is in range of 50-4000 µm; preferably, the diameter of circumcircle circle is in range of 50-1000 µm and the height of the needle bar 3 is in range of 200-2000 µm; the diameter of circumcircle at the bottom of the pyramid needle 4 is in line with the diameter of circumcircle of the pyramid connected with the pyramid needle 4, and the height of the pyramid needle 4 is in range of 50-2000 µm, preferably, 50-1000 µm; the radius of curvature of the circumcircle in the tip of the pyramid needle head 4 is in range of 50 nm-300 µm; preferably, less than 10 µm.

The needle bar 39 is cylinder, and the upper surface of the needle head 49 is a elliptic plane which has a setting acute angle with the plane of the substrate 2; the needle bar 310 is cylinder, and the needle head 410 has two elliptic planes which have a setting acute angle with the plane of the substrate 2; similarly, when the needle bar 3 is cylinder, the needle head 4 is a tip-like structure constituted of multiple elliptic or sectorial planes which have a setting acute angle with the plane of the substrate 2; the diameter of cross-section circular of the needle bar 39 and 310 is in range of 20-3000 µm and the height of the needle bar 39 and 310 is in range of 50-3000 µm respectively; preferably, the diameter of the cross-section circular is in range of 50-1000 µm and the height of the needle bar 39 and 310 is in range of 200-2000 µm respectively; the diameter of the bottom of the needle head 49 and 410 is in line with the diameter of the cross-section circular of the needle bar 39 and 310 respectively, and the height is in range of 50-2000 µm, preferably, 50-1000 µm; the thickness of blade of the tip of the needle head 4 is in range of 50 nm-300 µm, preferably, less than 10 µm.

The needle bar 3 and the needle 4 is constituted of prismoid and pyramid with more sides; the body of the micro-needle is any other structures with the tip-like shape.

As shown in Fig. 3, the axis of the needle bar 3 and the needle head 4 is vertical to or inclined at an angle toward the plane of the substrate 2; preferably, the axis of the needle bar 3 and the needle head 4 is vertical to the plane of the substrate 2;

The polymer micro-needle consisting the micro-needle array 1 is a solid micro-needle or a hollow micro-needle. As shown in Fig. 4, the hollow micro-needle is consisted of the needle bar 3, the needle head 4 and the inner hole 5; similarly, as shown in Fig. 2-1 to Fig. 2-5, the various types of solid micro-needle is processed into the hollow micro-needle with the inner hole; preferably, the polymer micro-needle is a solid micro-needle.

The spatial arrangement of the micro-needle array 1 is linear; is rectangular, square, parallelogram, circle, oval, triangle, or other graphics as shown in Fig. 5.

The micro-needle array 1 is a solid micro-needle array 1 or a hollow micro-needle array 1, or a mixture of both arrays; preferably, the micro-needle array 1 is a solid micro-needle array 1.

The thickness of substrate 2 of the micro-needle array chip is in range of 50-5000 µm, preferably, 100-2000 µm.

The spatial parameters of the micro-needle array chip comprise the shape, length, spacing and number of the micro-needle, the arrangement of the micro-needle array 1, and the thickness of the substrate 2 or the like which can be adjusted according to the needs.

The method for preparing the polymer micro-needle array chip as shown in Fig. 6, mainly comprising the following steps:

F-01, a prototype of micro-needle array chip with the same spatial features of the target polymer micro-needle array chip is prepared;

The prototype of micro-needle array chip is prepared by means of micro electro mechanical systems, such as numerical control laser processing or electrical discharge machining; and the material is any one selected from the group consisting of titanium, copper, aluminum, nickel, tungsten, stainless steel, titanium alloy, nickel alloy, aluminum alloy, copper alloy or other metals and alloy;

According to the differences of spatial features, the prototype of micro-needle array chip is divided into three types of A, B and C;

The prototype of type A micro-needle array chip has the same spatial features with the micro-needle array chip as shown in Fig. 1, which comprises a micro-needle array 1 and a substrate 2;

The shortest distance between the cross-section circular and the outside of the upper surface of the substrate 2 is in range of 100-5000 µm, with the cross-section circular on the border of the outermost micro-needles of the prototype of type A micro-needle array chip and the substrate, preferably, 500-2000 µm;

As shown in Fig. 7, multiple prototypes of type A micro-needle array chip rank on the same smooth flat base surface 6 according to arbitrary topology;

The base 6 is made of material which is any one selected from the group consisting of titanium, copper, aluminum, nickel, tungsten, stainless steel, titanium alloy, nickel alloy, aluminum alloy, copper alloy or other metals and alloy; or is made of material such as glass, silicon, silicon dioxide and the like.

The spatial parameters of the prototype of type A micro-needle array comprise the number, height, spacing of the micro-needle, the angle formed by the micro-needle with the plane of the substrate 2, the arrangement of the micro-needle array 1, and the thickness of the substrate 2 or the like which can be adjusted according to the needs of the target polymer micro-needle array chip.

The prototype of type B micro-needle array chip shown in Fig. 8 comprises a micro-needle array 1, a substrate 2 and a concave ring 7 surrounding the substrate 2, which is formed on the base surface 6 directly;

The shortest distance between the cross-section circular and the outside of the upper surface of the substrate 2 is in range of 100-5000 µm, with the cross-section circular on the border of the outermost micro-needles of the prototype of type B micro-needle array chip and the substrate, preferably, 500-2000 µm;

The cross section of concave ring 7 of the prototype of type B micro-needle array can be rectangular, square or semi-circular and other regular or irregular shapes; the maximum depth of the cross section of concave ring 7 is in range of 100-5000 µm and the maximum width is in range of 100-5000 µm; preferably, the maximum depth of the cross section of concave ring 7 is in range of 100-2000 µm and the maximum width is in range of 100-2000 µm;

For the prototype of type B micro-needle array chip, one micro-needle array 1, substrate 2 and corresponding concave ring 7 can be separately formed on the base surface 6 every time, or multiple micro-needle array 1, substrate 2 and corresponding concave ring 7 can be formed on the same base surface 6; multiple micro-needle array 1, substrate 2 and corresponding concave ring 7 can rank on the base surface 6 according to arbitrary topology;

The spatial parameters of the prototype of type B micro-needle array comprise the number, height, spacing of the micro-needle, the angle formed by the micro-needle with the plane of the substrate, the thickness of the substrate, the arrangement of the micro-needle array 1, the substrate 2 and the corresponding concave ring 7 on the base surface 6 or the like, which can be adjusted according to the needs of the target polymer micro-needle array chip.

The prototype of type C micro-needle array chip shown in Fig. 9 comprises a micro-needle array 1, a substrate 2, a concave ring 7 around the substrate 2 and a side 8 vertical to the substrate 2 and around the concave ring 7, which is formed on the base surface 6 directly;

In the prototype of type C micro-needle array chip, the shortest distance between the cross-section circular and the outside of the upper surface of the substrate 2 is in range of 100-5000 µm, with the cross-section circular on the border of the outermost micro-needles and the substrate, preferably, 500-2000 µm;

The cross section of concave ring 7 of the prototype of type C micro-needle array can be rectangular, square or semi-circular and other regular or irregular shapes; the maximum depth of the cross section of concave ring 7 is 100-5000 µm and the maximum width is 100-5000 µm; preferably, the maximum depth of the cross section of concave ring 7 is in range of 100-2000 µm and the maximum width is in range of 100-2000 µm;

In the prototype of type C micro-needle array chip, the vertical distance between any position of the outside of concave ring 7 and the side 8 is in range of 0-5000 µm; preferably, the vertical distance is 0 µm, namely, the outside of concave ring 7 overlaps with the inside of side 8;

The vertical height between the upper surface of the side 8 and the upper surface of the substrate 2 is in range of 50-5000 µm higher than that between the tip of the needle head 4 and the upper surface of the substrate 2; preferably, the difference in vertical height is in range of 200-2000 µm;

In the prototype of type C micro-needle array chip, one micro-needle array 1, substrate 2, corresponding concave ring 7 and side 8 can be separately formed on the base surface 6 every time, or multiple micro-needle array 1, substrate 2, corresponding concave ring 7 and side 8 can be formed on the same base surface 6 every time; multiple micro-needle array 1, substrate 2, corresponding concave ring 7 and side 8 can rank on the base surface 6 according to arbitrary topology;

The spatial parameters of the prototype of type C micro-needle array comprise the number, height, spacing of the micro-needle, the angle formed by the micro-needle with the plane of the substrate, the thickness of the substrate, the arrangement of the micro-needle array 1, the substrate 2, corresponding concave ring 7 and the side 8 on the base surface 6 or the like, which can be adjusted according to the needs of the target polymer micro-needle array chip;

F-02, the cavity die is prepared by using the prototypes of micro-needle array chip:

As shown in Fig. 10, when the cavity die is prepared by using the prototypes of the type A micro-needle array chip, single or multiple prototypes of the micro-needle array chip prepared in F-01 is placed on the base surface 6; then a closed side wall 8 is constructed around every prototype of micro-needle array chip, which is vertical to the base surface 6 and surrounds the prototype of the micro-needle array chip; finally, a open-top three-dimensional structure is formed by a base surface 6 and closed side wall 8;

When the cavity die is prepared by using the prototypes of the type A micro-needle array chip, the side 8 is made of material which is any one selected from titanium, copper, aluminum, nickel, tungsten, stainless steel, titanium alloy, nickel alloy, aluminum alloy, copper alloy or other metals and alloy; or is made of material which is any one selected from such as glass, silicon, silicon dioxide and the like.

When the cavity die is prepared by using the prototypes of the type A micro-needle array chip, the shortest distance between any position of the inside on the border of the side 8 and the base surface 6 and the outermost of the border of the substrate 2 and the base surface 6 is in range of 100-5000 µm; preferably, 500-2000 µm;

When the cavity die is prepared by using the prototypes of the type A micro-needle array chip, the vertical height between the upper place of the side 8 and the upper place of the base surface 6 is in range of 50-5000 µm higher than that of the sum of the substrate 2 of the prototypes of the micro-needle array chip and the micro-needle; preferably, 100-2000 µm;

When the cavity die is prepared by using the prototypes of the type A micro-needle array chip, the polymer in liquid or molten state is poured into and filled up the three-dimensional structure from the opening, after the completion of the preparation of the three-dimensional structure; the polymer is at least one or more selected from polyethylene, polypropylene, polylactic acid, poly(butylene succinate) and polydimethylsiloxane or other polymers; preferably, the polymer in liquid or molten state for pouring should be easy for demoulding; more preferably, the cured polymer has a hardness suitable for next steps;

When the cavity die is prepared by using the prototypes of the type B micro-needle array chip, then a closed side wall 8 is constructed which is vertical to the base surface 6 and surrounds the concave ring 7, finally, a open-top three-dimensional structure is formed with the base surface 6 and the closed side wall 8, as shown in Fig. 9;

When the cavity die is prepared by using the prototypes of the type B micro-needle array chip, the side 8 is made of material which is any one selected from the group consisting of titanium, copper, aluminum, nickel, tungsten, stainless steel, titanium alloy, nickel alloy, aluminum alloy, copper alloy or other metals and alloy; or is made of material which is any one selected from the group consisting of glass, silicon, silicon dioxide and the like.

When the cavity die is prepared by using the prototypes of the type B micro-needle array chip, the shortest distance between any position of the inside on the border of the side 8 and the base surface 6 and the outside of the concave ring 7 is in range of 0-5000 µm; preferably, the shortest distance is 0 µm, namely, the outside of concave ring 7 overlaps with the inside of side 8;

When the cavity die is prepared by using the prototypes of the type B micro-needle array chip, the vertical height between the upper place of the side 8 and the upper place of the substrate 2 is in range of 50-5000 µm higher than that between the top of the micro-needle head 4 and the upper place of the substrate 2; preferably, 100-2000 µm;

When the cavity die is prepared by using the prototypes of the type B micro-needle array chip, the polymer in liquid or molten state is poured into and filled up the three-dimensional structure from the opening, after the completion of the preparation of the three-dimensional structure; the polymer is at least one or more selected from polyethylene, polypropylene, polylactic acid, poly (butylene succinate) and polydimethylsiloxane or other polymers; preferably, the polymer in liquid or molten state for pouring should be easy for demoulding; more preferably, the cured polymer has a hardness suitable for next steps;

When the cavity die is prepared by using the prototypes of the type C micro-needle array chip, the polymer in liquid or molten state is poured into and filled up the three-dimensional structure from the opening; the polymer is at least one or more selected from polyethylene, polypropylene, polylactic acid, poly (butylene succinate) and polydimethylsiloxane or other polymers; preferably, the polymer in liquid or molten state for pouring should be easy for demoulding; more preferably, the cured polymer has a hardness suitable for next steps;

F-03, the prototype of micro-needle array chip is demoulded to obtain the cavity die

Demoulding the prototype F-03, after the polymer solution or molten polymer injected into the mould in the step F-02 is cured. As shown in Fig. 11, there is single cavity dies in the polymer template 9, comprising a micro-needle chamber 10 and a substrate chamber 11. As shown in Fig.12, there are multiple cavity dies in the polymer template 9, every cavity die is consisted of a micro-needle chamber 10 and a substrate chamber 11;

F-04, the micro-needle array chip is prepared using cavity die

According to the difference of the types of materials used by the micro-needle array 1 and the substrate 2 of the polymer micro-needle array chip, the method for preparing micro-needle array chip using the polyacrylamides polymer or the mixture composed of the bioactive substances or drugs is divided into three types of F1, F2 and F3.

F1-type method for preparing polymer micro-needle array chip:

The F1-type method refers to that the same type of material is used for preparing the micro-needle array 1 and the substrate 2 when the polymer micro-needle array chip is prepared.

The same type of material refers to the polyacrylamides polymer or the mixture of the polyacrylamides polymer and the bioactive substances or drugs. The bioactive substances or drugs is one or more selected from the group consisting of vaccines, polypeptides, proteins, polysaccharides, nucleic acids, hormones, anti-cancer drugs, genetic engineering drugs, natural product drugs, the traditional Chinese medicine or nutrients with any molecular weight;

The specific step is as follows:
1, if the material for preparing is a mixture of the polyacrylamides polymer and the bioactive substances or drugs, the polyacrylamides polymer is needed to be mixed with the bioactive substances or drugs in solid or liquid state in a certain proportion; the bioactive substances or drugs is present in the mixture composed of the bioactive substances or drugs and the polyacrylamides polymer in amount of 0.1-50 mass%; preferably, 10-20 mass%, to ensure the mechanical strength of the micro-needle can easily pierce the skin; the specific mixing ratio of the polyacrylamide polymer and the bioactive substances or drugs can be adjusted according to the dosage required for the treatment of diseases and the spatial feature of the polymer micro-needle array chip prepared;
2, the polyacrylamides polymer or the mixture composed of the polyacrylamide polymer and the bioactive substances or drugs, is mixed with water to obtain aqueous solution or mixture solution at the temperature in range of 10-90°C; the polyacrylamides polymer or the mixture composed of the polyacrylamide polymer and the bioactive substances or drugs is present in the aqueous solution or mixture solution in amount of 1-80 mass%; preferably, 10-50 mass%; preferably, the above mixture solution is processed by ultrasonic wave to remove bubbles.
3, the mould prepared in step F-03 is cleaned with water and then placed on a horizontal operation platform; preferably, the mould and the horizontal operation platform is placed in a closed system to ensure that the preparation of polymer micro-needle array chip is not affected by the environment; preferably, the horizontal operation platform and the mould are sealed with viscous liquid to ensure that mould is tightly fixed on the horizontal operation platform, while ensuring that the mould can be easily removed from the platform when the experiment is finished;
4, the aqueous solution or mixture solution prepared in step 2 is poured into the cavity die prepared in F-03 and as shown in Fig. 11 or 12; the volume of the aqueous solution or mixed liquor poured is determined by the volume of the micro-needle array chamber 10, the volume of the substrate chamber 11, and the mass fraction of the polyacrylamides polymer or the mixture composed of the polyacrylamide polymer and the bioactive substances or drugs in the aqueous solution or mixed liquor;
5, drying the aqueous solution or mixture solution poured into the cavity die in step 4 at the temperature in range of 20-90°C; preferably, 20-50°C; in case that the aqueous solution or the mixture of the mixture solution for a once-through pour in the cavity die is sufficient to prepare the polymer micro-needle array chip, drying the aqueous solution or the mixture solution in the cavity die directly and curing the polyacrylamides polymer or the mixture to obtain the polymer micro-needle array chip as designed;
in case that the aqueous solution or the mixture of the mixture solution for a once-through pour in the cavity die is not sufficient to prepare the polymer micro-needle array chip, drying the aqueous solution or the mixture solution in the cavity die to remove part of the water therein; pouring for a second time or more times until the polyacrylamides polymer or the mixture in the cavity die is sufficient to prepare the polymer micro-needle array chip; drying and curing the polyacrylamides polymer or the mixture to obtain the polymer micro-needle array chip as designed.

F2-type method for preparing polymer micro-needle array chip:

The F2-type method refers to that the different types of material are used for preparing the micro-needle array 1 and the substrate 2 when the polymer micro-needle array chip is prepared.

The micro-needle body 1 and the substrate film with the thickness less than 50 µm which connects with the micro-needle body 1 is made of the mixture composed of the polyacrylamide polymer and the bioactive substances or drugs; the substrate body is made of the polyacrylamides polymer. The bioactive substances or drugs is one or more selected from the group consisting of vaccines, polypeptides, proteins, polysaccharides, nucleic acids, hormones, anti-cancer drugs, genetic engineering drugs, natural product drugs, the traditional Chinese medicine or nutrients with any molecular weight;

The specific step is as follows:
1, the polyacrylamides polymer is mixed with the bioactive substances or drugs in solid or liquid state in a certain proportion; the bioactive substances or drugs is present in the mixture composed of the bioactive substances or drugs and the polyacrylamides polymer in amount of 0.1-50 mass%; preferably, 10-20 mass%, to ensure the mechanical strength of the micro-needle to easily pierce the skin; the specific mixing ratio of the polyacrylamides polymer and the bioactive substances or drugs can be adjusted according to the dosage required for the treatment of diseases and the spatial features of the polymer micro-needle array chip prepared;
2, the mixture composed of the polyacrylamides polymer and the bioactive substances or drugs is mixed with water to obtain mixture solution at the temperature of 10-90°C; the mixture composed of the polyacrylamide polymer and the bioactive substances or drugs is present in the mixture solution in amount of 1-80 mass%; preferably, 10-50 mass%; preferably, the above mixture solution is processed by ultrasonic wave to remove bubbles.
3, the cavity die prepared in step F-03 is cleaned with water and then placed on a horizontal operation platform; preferably, the mould and the horizontal operation platform is placed in a closed system to ensure that the preparation of polymer micro-needle array chip is not affected by the external environment; preferably, the horizontal operation platform and the mould are sealed with viscous liquid to ensure that mould is tightly fixed on the horizontal operation platform, while ensuring that the mould can be easily removed from the platform when the experiment is finished;
4, the aqueous solution or mixture solution prepared in step 2 is poured into the cavity die prepared in F-03 and as shown in Fig. 11 or 12; the volume of the aqueous solution or mixture solution poured is determined by the volume of the micro-needle array chamber 10 and the mass fraction of the mixture composed of the polyacrylamides polymer and the bioactive substances or drugs in the aqueous solution or mixture solution;
5, the aqueous solution or mixture solution poured into the cavity die in step 4 is dried at the temperature in range of 20-90°C; preferably, 20-50°C; if the aqueous solution or the mixture composed of the polyacrylamides polymer and the bioactive substances or drugs in the mixed liquor, poured into the cavity die for one-time can meet the needs of the preparation of the micro-needle array 1 and the substrate 2 film with the thickness less than 50 µm which connects with the micro-needle array 1, the aqueous solution or the mixture solution poured into the cavity die can be dried directly to cure the mixture composed of the polyacrylamides polymer and the bioactive substances or drugs therein to obtain the micro-needle array 1 and the substrate 2 film with the thickness less than 50 µm which connects with the micro-needle array 1 of the micro-needle array chip complying with the design requirements; if the aqueous solution or the mixture composed of the polyacrylamides polymer and the bioactive substances or drugs in the mixture solution, poured into the cavity die for one-through can't meet the needs of the preparation of the micro-needle array 1 and the substrate 2 film with the thickness less than 50 µm which connects with the micro-needle array 1, the aqueous solution or the mixture solution poured into the cavity die can be dried to remove parts of the water therein. Then, pouring for a second or more times is conducted until the mixture composed of the polyacrylamides polymer and the bioactive substances or drugs can meet the needs of the preparation of the micro-needle array 1 and the substrate 2 film with the thickness less than 50 µm which connects with the micro-needle array 1. Finally, curing the mixture composed of the polyacrylamide polymer and the bioactive substances or drugs by drying to obtain the micro-needle array 1 and the substrate 2 film with the thickness less than 50 µm which connects with the micro-needle array 1 of the micro-needle array chip complying with the design requirements.
6, the polyacrylamides polymer is mixed with water to obtain aqueous solution at the temperature in range of 10-90°C; the polyacrylamide polymer is present in the aqueous solution or mixture solution in amount of 20-80 mass%; preferably, 40-60 mass%; preferably, the polyacrylamides polymer of the aqueous solution poured into the cavity die for one-through can meet the needs of the preparation of the substrate body of the micro-needle array chip; preferably, the above mixture solution is processed by ultrasonic wave to remove bubbles.
7, the aqueous solution of the pure polyacrylamides polymer prepared in step 6 is poured into the cavity die used in step 3-5; the volume of the aqueous solution of the polymer is determined by the partial volume of the substrate chamber 11 and the mass fraction of the polyacrylamides polymer in the aqueous solution;
8, the aqueous solution poured into the cavity die in step 7 is dried at the temperature in range of 20-90°C; preferably, 20-50°C; by drying, curing the polyacrylamide polymer poured into the cavity die to obtain the substrate body of the micro-needle array chip and then obtain the entire polymer micro-needle array chip;

F3-type method for preparing polymer micro-needle array chip:

The F3-type method refers to that the different types of material are used for preparing the micro-needle array 1 and the substrate 2 when the polymer micro-needle array chip is prepared.

The micro-needle array 1 and the substrate film with the thickness less than 50 µm which connects with the micro-needle array 1 is made of the polyacrylamides polymer or the mixture composed of the polyacrylamide polymer and the bioactive substances or drugs; the substrate film is made of a material selected from the group consisting of polyethylene, polypropylene, poly(butylene succinate), polydimethylsiloxane, rubber, polylactic acid, latex and other medical polymers, glass and metal thermoplastic composite materials;

The specific step is as follows:
1, if the material is the mixture of polyacrylamides polymer and the bioactive substances or drugs, the polyacrylamides polymer is needed to be mixed with the bioactive substances or drugs in solid or liquid state in a certain proportion; the bioactive substances or drugs is present in the mixture composed of the bioactive substances or drugs and the polyacrylamides polymer in amount of 0.1-50 mass%; preferably, 10-20 mass%, to ensure the mechanical strength of the micro-needle can easily pierce the skin; the specific mixing ratio of the polyacrylamides polymer and the bioactive substances or drugs can be adjusted according to the dosage required for the treatment of diseases and the spatial feature of the polymer micro-needle array chip prepared;
2, the polyacrylamide polymer or the mixture composed of the polyacrylamide polymer and the bioactive substances or drugs, is mixed with water to obtain aqueous solution or mixture solution at the temperature in range of 10-90°C; the polyacrylamide polymer or the mixture composed of the polyacrylamide polymer and the bioactive substances or drugs is present in the aqueous solution or mixture solution in amount of 1-80 mass%; preferably, 10-50 mass%; preferably, the above mixture solution is processed by ultrasonic wave to remove bubbles.
3, the mould prepared in step F-03 is cleaned with water and then placed on a horizontal operation platform; preferably, the mould and the horizontal operation platform is placed in a closed system to ensure that the preparation of polymer micro-needle array chip is not affected by the external environment; preferably, the horizontal operation platform and the mould are sealed with viscous liquid to ensure that mould is tightly fixed on the horizontal operation platform, while ensuring that the mould can be easily removed from the platform when the experiment is finished;
4, the aqueous solution or mixture solution prepared in step 2 is poured into the cavity die prepared in F-03 and as shown in Fig. 11 or 12; the volume of the aqueous solution or mixture solution poured is determined by the volume of the micro-needle array chamber 10 and the mass fraction of the polyacrylamides polymer or the mixture composed of the polyacrylamide polymer and the bioactive substances or drugs in the aqueous solution or mixture solution;
5, the aqueous solution or mixture solution poured into the cavity die in step 4 is dried at the temperature in range of 20-90°C; preferably, 20-50°C;
   in case that the aqueous solution or the mixture of the mixture solution for a once-through pour in the cavity die is sufficient to prepare the polymer micro-needle array 1 and the substrate film with the thickness less than 50 µm connected thereto of the polymer micro-needle array chip, drying the aqueous solution or the mixture solution in the cavity die directly and curing the mixture to obtain the polymer micro-needle array 1 and the substrate film with the thickness less than 50 µm connected thereto of the polymer micro-needle array chip;
   in case that the aqueous solution or the mixture of the mixture solution for a once-through pour in the cavity die is not sufficient to prepare the polymer micro-needle array 1 and the substrate film with the thickness less than 50 µm connected thereto of the polymer micro-needle array chip, drying the aqueous solution or the mixture solution in the cavity die to remove part of the water therein; pouring for a second time or more times until the mixture in the cavity die is sufficient to prepare the polymer micro-needle array 1 and the substrate film with the thickness less than 50 µm connected thereto of the polymer micro-needle array chip; drying the mixture solution and curing the mixture to obtain the polymer micro-needle array 1 and the substrate film with the thickness less than 50 µm connected thereto of the polymer micro-needle array chip, as designed.
6, the one or more film material consisting the substrate body is combined tightly with the micro-needle array 1 and the substrate film with the thickness less than 50 µm which connects with the micro-needle array 1 by cohering, fusion, bonding or other physical or chemical methods, to obtain the entire polymer micro-needle array chip; the other one or more film material is a combination selected from the group consisting of polyethylene, polypropylene, poly (butylene succinate), polydimethylsiloxane, rubber, polylactic acid, latex and other medical polymers, glass and metal thermoplastic composite materials;

F-05, the preservation of the micro-needle array chip

The polymer micro-needle array chip can be removed from cavity die and preserved in a appropriate instrument or continues to be preserved in the cavity die; preferably, the polymer micro-needle array chip continues to be preserved in the cavity die, for the preparation of a patch for micro-needle transdermal drug delivery.

A patch for polymer micro-needle transdermal drug delivery prepared by using polymer micro-needle array chip as shown in Fig. 13, comprises a micro-needle array chip composed of a substrate 2 and a micro-needle array 1 standing thereon, also comprises base plate 12, anti-seepage washer 13, anti-adhesion layer 14, adhesive tape 15 and anti-seepage layer 16; the base plate 12 is adhesive to the back of the substrate 2, the anti-seepage washer 13 surrounds the edge of the substrate 2 and the base plate 12; the anti-adhesion layer 14 covers the outside of the anti-seepage washer; the adhesive tape 15 is a double sided sticky tape which is sticky on both sides, one side of the tape covers the base plate 12, the anti-seepage washer 13 and the anti-adhesion layer 14, the other side of the tape is adhered to the anti-seepage layer 16; the outside of the adhesive tape 15 is covered by anti-seepage layer 16;

The base plate 12 of the present invention is one or more film adhere to the back of micro-needle array chip, which can be prepared using at least one selected from the group consisting of plastic, polymer, synthetic resin, latex, rubber, glass, ceramic, metal or composite materials; it can be one or more film; when it is more films structure, every film can be combined together tightly by cohering, fusion, bonding and physical methods; preferably, at least one film composed of base plate 12 in more films is hard film; the size of substrate 12 is equal to or larger than the size of substrate 2 of micro-needle array chip; the base plate 12 is mainly used to protect the micro-needle array chip to avoid influence of the external environment and contribute to the micro-needle array piercing the stratum corneum of skin easily; a patch for micro-needle transdermal drug delivery of the present invention can comprise base plate 12, or not comprise base plate 12; preferably, when substrate 2 of micro-needle array chip is prepared using polyacrylamides polymer or a mixture of polyacrylamides polymer with the bioactive substances or drugs, the back of substrate 2 of micro-needle array chip is adhered with base 12, when substrate 2 of micro-needle array chip is prepared using other material, especially insoluble material, the back of substrate 2 is not adhered to base plate 12;

The anti-seepage washer 13 of the present invention surrounds the base plate 12 of the patch for micro-needle transdermal drug delivery, to protect the micro-needle array chip to avoid influence of the external environment, especially incursion of aqueous solution; it can be prepared by one or more selected from the group consisting of latex, rubber, medical plastic, polymer; the shape of anti-seepage washer 13 is determined by the shape of base plate 12 of the patch for micro-needle transdermal drug delivery surrounding, namely, the inside shape of anti-seepage washer 13 is consistent with the outside shape of base plate 12; the cross-section of anti-seepage washer 13 can be rectangle, roundness and other shapes. Preferably, the cross-section of anti-seepage washer 13 is rectangle; the height of cross-section of anti-seepage washer 13 is slightly larger than or equal to the height of base plate 12 of patch for micro-needle transdermal drug delivery; preferably, the height of anti-seepage washer 13 is equal to the height of base plate 12; the width of cross-section of anti-seepage washer 13 is in range of 20-5000 µm; preferably, the width of cross-section of anti-seepage washer 13 is in range of 200-2000 µm; the patch for micro-needle transdermal drug delivery of the present invention can comprise a anti-seepage washer 13, or not comprise anti-seepage washer 13; preferably, if a patch for micro-needle transdermal drug delivery comprises base plate 12, the base plate 12 is surrounded with the anti-seepage washer 13, if a patch for micro-needle transdermal drug delivery does not comprise base plate 12, it can't comprise anti-seepage washer 13.

The anti-adhesion layer 14 of the present invention is a thin film covering the outside of anti-seepage washer of patch for micro-needle transdermal drug delivery, prepared by using the material does not adhere to adhesive tape 15; when the drugs are used, the anti-adhesion layer 14 is easy to be peeled off, and it is convenient for adhesive tape 15 to fix patch for micro-needle transdermal drug delivery; the area and shape of anti-adhesion layer 14 is determined by the outside of adhesive tape 15; as is shown in Fig. 13, the size and the area of adhesive tape 15 is the sum of the area of base plate 12, anti-seepage washer 13 and anti-adhesion layer 14; if a patch for micro-needle transdermal drug delivery does not comprise base plate 12 and anti-seepage washer 13, the area of adhesive tape 15 is the sum of the area of the substrate 2 and the anti-adhesion layer 14;

The one side of the adhesive tape 15 covers the base plate 12, the anti-seepage washer 13 and the anti-adhesion layer 14, the other side of the tape is adhered to the anti-seepage layer 16. When drugs are used, the anti-adhesion layer 14 is peeled off, the adhesive tape 15 can fix the patch for micro-needle transdermal drug delivery on the skin surface; the adhesive tape can be any shape; preferably, the adhesive tape is rectangle. The length of adhesive tape is in range of 1-150 mm and the width is in range of 1-100 mm. Preferably, the length is in range of 10-50mm and the width is in range of 5-30 mm;

The anti-seepage layer 16 of the present invention is a protective film covering the outside of the adhesive tape 15, and the size and shape are equal to or slightly larger than the adhesive tape 15; the anti-seepage layer 16 is mainly used to prevent aqueous solution inrushing into polymer micro-needle array chip, and can be prepared by using one or more mixture selected from the group consisting of hydrophobic various kinds of fiber, latex, rubber and the like;

Before used, the micro-needle array chip usually needs to be placed in the protective device to avoid the broken of the micro-needle for the external force; preferably, after prepared, the micro-needle array chip continues to be preserved in the mould as shown in Fig. 11 and 12. The mould preparing the micro-needle array chip comprises template 9, micro-needle cavity 10 and substrate cavity 11. Furthermore, preferably, when a patch for the micro-needle transdermal drug delivery is prepared and preserved, the micro-needle array chip always is preserved in the mould and not taken out until a patch for the micro-needle transdermal drug delivery is used for the treatment;

The present invention provides a method for preparing a patch for the micro-needle transdermal drug delivery, comprising the following steps:
1.Base plate 12 is adhered to substrate 2 of micro-needle array chip together: specifically, when the back of the substrate 2 of the micro-needle array chip is adhered to base plate 12, if base plate 12 is one film, it can be combined together with substrate 2 by cohering, fusion, bonding or physical methods; preferably, substrate 2 and base plate 12 is combined together by cohering; if structure of the base plate was more films, the more films can be adhered to each other, and then base plate 12 with more films is adhered to substrate 2 of micro-needle array chip together; the more films can be adhered to the back of substrate 2 of micro-needle array chip gradually according to a certain order; preferably, base plate 12 with more films structure is firstly prepared, and then it is adhered to substrate 2 of micro-needle array chip; if the back of substrate 2 does not need to be adhered to base plate 12, the method for preparing does not comprise this step;
2. An anti-seepage washer 13 is fixed around the base plate 12 of a patch for micro-needle transdermal drugs delivery; specifically, the inner shape of the anti-seepage washer is the same as the shape base plate 12, and the anti-seepage washer 13 is fixed around the base plate 12 by physical methods; if the anti-seepage washer 13 is not contained in the patch for micro-needle transdermal drugs delivery, this step is omitted;
3. The anti-adhesion layer 14 is placed on the outside of base plate 12 or substrate 2 of micro-needle array chip; specifically, according to the shape and area of adhesive tape 15 as well as anti-seepage washer 13, anti-adhesion layer 14 is pre-prepared, and then the prepared anti-adhesion layer 14 is placed on the outside of anti-seepage washer 13; if the base plate 12 and anti-seepage washer 13 are not contained in the patch for micro-needle transdermal drugs delivery, the anti-adhesion layer 14 is pre-prepared according to the shape and area of adhesive tape 15 and substrate 2, and then the prepared anti-adhesion 14 is placed at the outside of substrate 2;
4. An adhesive tape 15 is adhered to anti-seepage layer 16 together; specifically, according to the shape and area of adhesive tape 15, anti-seepage layer 16 is pre-prepared to have the same shape and area as adhesive tape 15, and then adhered together; or an adhesive tape 15 can be adhered to anti-adhesion layer 16 together, and be cut to the required shape and area;
5. The one side of adhesive tape 15 covers the base plate 12, the anti-seepage washer 13 and the anti-adhesion layer 14; specifically, adhesive tape 15 which is adhered to anti-seepage layer 16 covers the base plate 12, anti-seepage washer 13 and anti-adhesion layer 14 of the patch for micro-needle transdermal drugs delivery, and be adhered to together. If micro-needle transdermal drugs delivery patch didn't contain base plate 12 and anti-seepage washer 13, the one side of adhesive tape 15 is adhered together to anti-adhesion layer 14;

The order of step F-04 and F-05 can be adjusted according to the actual situation.

Next, in order to illustrate the present invention better, some examples are provided in detail.

Examples 1-14 are provided to illustrate the method of synthesizing a medical water soluble polyacrylamides polymer using acrylamide monomer. Polyacrylamides polymer obtained by synthesis can be used for preparing medical poly micro-needle array chip.

Examples 1-3 are provided to illustrate the effect of the ratio of water and a variety of organic solvents on the reaction of polymerization of acrylamide.

### Example 1

According to the volume ratio of 2:5:10:83, 100 mL of water, ethanol, acetone and isopropanol were added to in turn three necked round bottom flask with the 250 mL comprising a mixing flow condenser, a thermometer and a nitrogen introducing device, using a magnetic stirrer to mix them uniformly; acrylamide monomer of 10.66 g was added to the system continuously with stirring to make it dissolved; nitrogen was added to the system, and the target temperature was set to 60°C with water bath heating; 107 mg of 2,2-azobisisobutyronitrile was dissolved in 10mL of isopropanol and mixed uniformly to dissolve thoroughly. 1mL of isopropanol with 2,2-azobisisobutyronitrile was added to the system when the temperature of the reaction system reached to the target temperature 60°C, kept the reaction for 15 hours with stirring and the high purity nitrogen introduced continuously;

After reaction, the reaction system was cooled and solid-liquid of the reaction system were separated using vacuum filtration, and then the solid product obtained was placed into vacuum drying box for drying at the temperature of 55°C; the dried product was dissolved in water until it was completely dissolved, and then the mixture solution comprising ethanol and acetone in volume ratio 6:4 was used to precipitate the polymer for removing unreacted monomer; repeating the dissolution, precipitation, filtration and drying three times, and keeping the resultant samples in dry and closed container.

The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 9.1×10⁴. According to the standard of GB/T4340.2, Vickers hardness of corresponding bulk material was about 430HV. According to the standard of D-256 of US ATSM, the impact strength was about 20J/m.

### Example 2

The method of example 1 was repeated except that the volume ratio of water, ethanol, acetone and isopropanol was 5:5:10:80. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 1.35×10⁵. According to the standard of GB/T4340.2, Vickers hardness of corresponding bulk material was about 300 HV According to the standard of D-256 of US ATSM, the impact strength was about 25 J/m.

### Example 3

The method of example 1 was repeated except that the volume ratio of water, ethanol, acetone and isopropanol was 10:5:10:80. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 2.0×10⁵. According to the standard of GB/T4340.2, Vickers hardness of corresponding bulk material was about 150 HV According to the standard of D-256 of US ATSM, the impact strength was about 30 J/m.

Examples 1, 4-6 were used to illustrate the effect of initial concentration of acrylamide monomer on the reaction of polymerization of acrylamide.

### Example 4

The method of example 1 was repeated except that the weight of acrylamide monomer was 3.56 g. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 6.3×10⁴. According to the standard of GB/T4340.2, Vickers hardness of corresponding bulk material was about 550HV. According to the standard of D-256 of US ATSM, the impact strength was about 9 J/m.

### Example 5

The method of example 1 was repeated except that the weight of acrylamide monomer was 7.11 g. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 7.6×10⁴. According to the standard of GB/T4340.2, Vickers hardness of corresponding bulk material was about 510 HV According to the standard of D-256 of US ATSM, the impact strength was about 14 J/m.

### Example 6

The method of example 1 was repeated except that the weight of acrylamide monomer was 14.22 g. The residual amount of acrylamide monomer in polymer was measured by SHIMADZU liquid chromatograph (LC-20A/SPD-20AV), the measured value was not more than 0.5ppm. According to GB 17514-2008 method and static light scattering method (Wyatt DAWN HELEOS-II), the molecular weight was measured; the measured value of molecular weight of obtained polymer was 1.2×10⁵. According to the standard of GB/T4340.2, Vickers hardness of corresponding bulk material was about 330 HV According to the standard of D-256 of US ATSM, the impact strength was about 24 J/m.

Examples 1, 7-9 were used to illustrate effect of temperature on the reaction of polymerization of acrylamide.

### Example 7

The method of example 1 was repeated except that the target temperature was 55°C. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 9.5×10⁴. According to the standard of GB/T4340.2, Vickers hardness of corresponding bulk material was about 410 HV According to the standard of D-256 of US ATSM, the impact strength was about 21 J/m.

### Example 8

The method of example 1 was repeated except that the target temperature was 65°C. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 8.7×10⁴. According to the standard of GB/T4340.2, Vickers hardness of corresponding bulk material was about 450 HV According to the standard of D-256 of US ATSM, the impact strength was about 19 J/m.

### Example 9

The method of example 1 was repeated except that the target temperature was 70°C. The residual amount of acrylamide monomer in polymer was measured by SHIMADZU liquid chromatograph (LC-20A/SPD-20AV), the measured value was not more than 0.5ppm. According to GB 17514-2008 method and static light scattering method (Wyatt DAWN HELEOS-II), the molecular weight was measured; the measured value of molecular weight of obtained polymer was 7.8×10⁴. According to the standard of GB/T4340.2, Vickers hardness of corresponding bulk material was about 490 HV According to the standard of D-256 of US ATSM, the impact strength was about 16 J/m.

Examples 1, 10-13 were used to illustrate effect of the amount of initiator on polymerization of acrylamide.

### Example 10

The method of example 1 was repeated except that the amount of initiator 2,2-azobisisobutyronitrile dissolved in 10 mL of isopropanol was 53.5 mg. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 1.6×10⁵. According to the standard of GB/T4340.2, Vickers hardness of corresponding bulk material was about 250 HV. According to the standard of D-256 of US ATSM, the impact strength was about 28 J/m.

### Example 11

The method of example 1 was repeated except that the amount of initiator 2,2-azobisisobutyronitrile dissolved in 10 mL of isopropanol was 214 mg. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 4.3×10⁴. According to the standard of GB/T4340.2, Vickers hardness of corresponding bulk material was about 600 HV. According to the standard of D-256 of US ATSM, the impact strength was about 10 J/m.

### Example 12

The method of example 1 was repeated except that the amount of initiator 2,2-azobisisobutyronitrile dissolved in 1 mL of isopropanol was 53.5 mg. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 1.0×10⁴. According to the standard of GB/T4340.2, Vickers hardness of corresponding bulk material was about 500 HV. According to the standard of D-256 of US ATSM, the impact strength was about 5 J/m.

Examples 1, 13-14 were used to illustrate effect of the kinds of initiator on polymerization of acrylamide.

### Example 13

The method of example 1 was repeated except that the initiator was changed to 107 mg ammonium persulfate and the solvent isopropanol used for dissolving initiator was 10 mL of water. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 7.7×10⁴. According to the standard of GB/T4340.2, Vickers hardness of corresponding bulk material was about 500 HV According to the standard of D-256 of US ATSM, the impact strength was about 15 J/m.

### Example 14

The method of example 1 was repeated except that the initiator was changed to 107 mg lauroyl peroxide and the solvent isopropanol used for dissolving initiator was 10 mL of water. The residual amount of acrylamide monomer in polymer was measured by SHIMADZU liquid chromatograph (LC-20A/SPD-20AV), the measured value was not more than 0.5 ppm. According to GB 17514-2008 method and static light scattering method (Wyatt DAWN HELEOS-II), the molecular weight was measured; the measured value of molecular weight of obtained polymer was 8.2×10⁴ Dalton. According to the standard of GB/T4340.2, Vickers hardness of corresponding bulk material was about 470 HV. According to the standard of D-256 of US ATSM, the impact strength was about 18 J/m.

### Example 15

This example was used to show the method of preparing type A micro-needle array chip prototype of present invention.

Nickel-Chromium stainless steel was used as material to prepare micro-needle array chip prototype; wire cutting by precision Electrical Discharge Machining, and polishing by Electrochemical Corrosion to obtain 8×8 micro-needle array chip prototype showing in Fig. 14; the needle bar 3 and the needle head 4 was a integrated pentagonal pyramid structure, diameter of circumscribed circle of the bottom of needle 3 on substrate 2 was 250 µm, the shortest distance of two circumscribed circles of the adjacent needle bars 3 on substrate 2 was 500 µm, the height of micro-needle was 1000 µm, radius of curvature of micro-needle tip was less than 10 µm, the thickness of substrate 2 of micro-needle array chip prototype was 500 µm; the shortest distance between any position of outside substrate 2 of micro-needle array chip prototype and micro-needle array 1 was 1000 µm; the spatial parameter of the above prototype of type A micro-needle array chip, including quantity, height, spacing, thickness of substrate, the shortest distance between the very outside substrate and micro-needle array and the like, could be adjusted according to the need;

The above prototype of type A micro-needle array chip could be prepared through the method of laser numerical control micro-processing and electrochemical corrosion;

The above prototype of type A micro-needle array chip could be prepared using any selected from the group consisting of titanium, copper, aluminum, nickel, tungsten, stainless steel, titanium alloy, nickel alloy, aluminum alloy and copper alloy and other metal or alloy through later numerical control micro-processing and electrochemical corrosion;

### Example 16

The example was used to illustrate the method of preparing the prototype of type B micro-needle array chip of the present invention.

Nickel-Chromium stainless steel was used as material to prepare the prototype of micro-needle array chip; wire cutting by precision Electrical Discharge Machining, and polishing by Electrochemical Corrosion to obtain the prototype of micro-needle array chip with 8×8 as shown in Fig.15; the needle bar 3 and the needle head 4 was a integrated triangular pyramid structure, diameter of circumscribed circle of the bottom of the needle 3 on the substrate 2 was 300 µm, the shortest distance of two circumscribed circles of adjacent needle bars 3 on substrate 2 was 750 µm, the height of micro-needle was 1200 µm, radius of curvature of micro-needle tip was less than 10 µm, the thickness of biological substrate 2 of micro-needle array chip prototype was 800um; the cross section of concave ring 7 around micro-needle array was rectangle, the height of the cross section of concave ring 7 was 500 µm, the width was 600 µm, the shortest distance between any position of inside surface of concave ring 7 and micro-needle array was 1000 µm;

The spatial parameter of the prototype of type B micro-needle array chip, including quantity, height, spacing, thickness of substrate 2, the height and width of concave ring 7, the shortest distance between any position of upper surface of the very outside of concave ring 7 and micro-needle array 1 of the micro-needle and the like, could be adjusted according to the need of the prototype of micro-needle array chip;

The prototype of type B micro-needle array chip could be prepared through the method of laser numerical control micro-processing and electrochemical corrosion;

The prototype of type B micro-needle array chip could be prepared using any selected from the group consisting of titanium, copper, aluminum, nickel, tungsten, stainless steel, titanium alloy, nickel alloy, aluminum alloy and copper alloy and other metal or alloy through laser numerical control micro-processing and electrochemical corrosion;

### Example 17

The example was used to show the method of preparing the prototype of type C micro-needle array chip of the present invention.

Nickel-Chromium stainless steel was used as material to prepare micro-needle array chip prototype; wire cutting by precision Electrical Discharge Machining, and polishing by Electrochemical Corrosion to obtain the prototype of micro-needle array chip with 8×8 as shown in Fig.16; micro-needle was a integrated rectangular pyramid structure containing a needle bar 3 and a needle head 4, diameter of circumscribed circle of the bottom of the needle 3 on the substrate 2 was 200 µm, the shortest distance of the circumscribed circles of the adjacent needle bars 3 on substrate 2 was 600 µm, the height of micro-needle was 900 µm, radius of curvature of micro-needle tip was less than 10 µm, the thickness of substrate of micro-needle array chip prototype was 1000 µm. The cross section of concave ring 7 around micro-needle array was rectangle. The height of the cross section of concave ring 7 was 600 µm, the width was 500 µm, the shortest distance between any position of the inside upper surface of concave ring 7 and micro-needle array 1 was 1000 µm; the outside of concave ring 7 overlaps the inside of the joint of the side wall 8 and the substrate 2. The vertical height from the upper surface of side 8 to the upper surface of substrate 2 was 500 µm higher than the vertical height from the micro-needle needle head 4 to the upper surface of substrate 2;

The spatial parameter of the prototype of type C micro-needle array chip, including quantity, height, spacing, thickness of substrate 2, the height and width of concave ring 7, the shortest distance between any position of upper surface the very outside of concave ring 7 and micro-needle array 1 of the micro-needle, the height of the side of and the like, could be adjusted according to the need of micro-needle array chip prototype;

The prototype of type C micro-needle array chip could be prepared through the method of laser numerical control micro-processing and electrochemical corrosion;

The prototype of type C micro-needle array chip could be prepared using any selected from the group consisting of titanium, copper, aluminum, nickel, tungsten, stainless steel, titanium alloy, nickel alloy, aluminum alloy and copper alloy and other metal or alloy through laser numerical control micro-processing and electrochemical corrosion;

### Example 18

This example was used to show the method of preparing cavity die through prototype of type A micro-needle array chip prepared in example 1; the specific steps were as follows:
1. As was shown in the Fig.10, a prototype of type A micro-needle array chip in example 1 was fixed on surface 6 of a glass, a closed side wall 8 made of glass surrounding the prototype of microneedle array and perpendicular to the surface 6, was formed onto the surface 6 of glass. Finally, an open-top three-dimensional structure is formed by the surface 6 and the closed side wall 8. The distance from the substrate 2 of prototype of micro-needle array chip to the joint of the wall 8 and the surface 6 was equal, which was 3000 µm in minimum. And the height of side wall 8 was 1500 µm;
2. An AB glue of polydimethylsiloxane in 10:1 was mixed uniformly by magnetic stirrer; the AB glue of polydimethylsiloxane was processed by ultrasonic wave to remove bubbles;
3. The AB glue of polydimethylsiloxane without bubbles was poured into the three-dimensional structure from the opening to fill up the three-dimensional structure;
4. The three-dimensional structure with the AB glue of polydimethylsiloxane was dried at 100°C for 5 hours.
5. The dried and cured AB glue of polydimethylsiloxane was taken out from the three-dimension structure, one or more cavity die using for preparing polymer micro-needle array chip as shown in Fig. 11 or 12 were obtained.

The glass plate for preparing the base 6 and the side wall 8 may be replaced by any one selected from the group consisting of titanium, copper, aluminum, nickel, tungsten, stainless steel, titanium alloy, nickel alloy, aluminum alloy and copper alloy and other metal or alloy;

The temperature for drying the AB glue of polydimethylsiloxane was in a range of 20-150 °C, the higher the drying temperature, the shorter the drying duration.

The AB glue of polydimethylsiloxane for pouring in the three-dimension structure may be replaced by one selected from the group consisting of polypropylene, polyethylene, polylactic acid and poly (butylene succinate) or other polymer in liquid state or molten state.

### Example 19

This example was used to show the method of preparing cavity die using prototype of type B micro-needle array chip prepared in example 2;

The specific steps were as follows:
1. The closed side wall 8 which was perpendicular to the substrate 2 and surrounds concave ring 7, was formed on the substrate using stainless steel, a open-top three-dimensional structure is formed by the substrate 2 and the closed side wall 8 (as shown in Fig. 9); the outside of concave ring 7 overlaps the inside of the joint of the side wall 8 and the substrate 2, the height of side wall was 1500 µm;
2. The molten polyethylene was poured into and filled up the three-dimensional structure from the opening; polyethylene in the three-dimensional structure was taken out when it was cooled, one or more cavity die using for preparing polymer micro-needle array chip as shown in Fig.11 or 12 were obtained.

The above stainless steel preparing side could be replaced by any selected from the group consisting of titanium, copper, aluminum, nickel, tungsten, stainless steel, titanium alloy, nickel alloy, aluminum alloy and copper alloy and other metal or alloy, also could be replaced by any selected from the group consisting of glass, silicon, silicon dioxide and other semiconductor materials;

The polyethylene in the three-dimension structure may be replaced by the one selected from the group consisting of polypropylene, polydimethylsiloxane, polylactic acid and poly (butylene succinate) or other polymer, in liquid or molten state.

### Example 20

This example was used to illustrate the method for preparing cavity die through the prototype of type C micro-needle array chip prepared in example 3;

The specific steps were as follows:

The molten polylactic acid was poured into and filled up the three-dimensional structure from the opening; the polylactic acid in the three-dimensional structure was taken out when it was cooled, one or more cavity die using for preparing polymer micro-needle array chip as shown in Fig. 11 or 12 were obtained.

The polylactic acid in the three-dimension structure may be replaced by the one selected from the group consisting of polypropylene, polyethylene, polydimethylsiloxane, polylactic acid and poly (butylene succinate) or other polymer, in liquid or molten state.

### Example 21

This example was used to show the method of preparing polymer micro-needle array chip using cavity die prepared in example 18-20;

The material used to prepare micro-needle array 1 and substrate 2 in the example was polyacrylamides polymer whose molecular weight was 1.0×10⁵;

The specific steps were as follows:
1. Polyacrylamides polymer was mixed with water according to 35:65 by mass ratio firstly, and then the polymer aqueous solution was processed by ultrasonic wave for 5 minutes to remove bubbles;
2. The polymer aqueous solution prepared in step 1, cavity die and horizontal operation platform prepared in examples 4-6 was placed in the glove box; lubricating oil was daubed on the surface of the horizontal operation platform, cavity die was fixed on the surface of the horizontal operation platform;
3. The polymer aqueous solution was poured into the cavity die as shown in Fig. 11 or 12; the volume of polymer aqueous solution preparing polymer micro-needle array chip was determined by the volume of chamber 10, substrate 11 of micro-needle array and the mass fraction of polymer in aqueous solution; the poured polymer aqueous solution was dried, the drying temperature was 40°C; if the polymer aqueous solution in cavity die could not meet the needs of the preparation of polymer micro-needle array chip, pouring for a second or more times was conducted after parts of the water therein could be dried to remove from the polymer aqueous solution. Finally, polymer of polymer aqueous solution poured in the cavity die was curing by drying to obtain the polymer micro-needle array chip meeting the design requirement.
4. The dried polyacrylamides polymer curing was taken out from the cavity die, polymer micro-needle array chip as shown in Fig. 17 were obtained.

Molecular weight of above polyacrylamides polymer was a certain value between 5.0×10⁴ and 2.0×10⁵; it also could be a mixture comprising different molecular weight which was between 5.0×10⁴ and 2.0×10⁵ according to a certain proportion;

The above polyacrylamides polymer in the aqueous solution was changed in range of 1-80 mass%;

The drying temperature could be changed in range of 30-90°C;

The drying duration could be adjusted according to temperature and other conditions.

### Example 22

The method of example 21 was repeated except that the material was changed to a mixture comprising polyacrylamides polymer and target drugs; the target drugs was bovine serum albumin, which was mixed with polyacrylamides polymer to use according to the mass ratio of 20:80;

The bovine serum albumin in the mixture comprising polyacrylamides polymer and bovine serum albumin was changed in range of 0.5-50 mass%;

The bovine serum albumin was replaced by one or a combination of several component selected from the group consisting of arbitrary molecular weight of vaccines, polypeptides, proteins, polysaccharides, nucleic acids, hormones, anti-cancer drugs, genetic engineering drugs, natural product drugs, the traditional Chinese medicine or nutrients.

### Example 23

This example was used to illustrate the method of preparing polymer micro-needle array chip using cavity die prepared in examples 18-20;

Micro-needle array 1 was prepared by using a mixture comprising polyacrylamides polymer and target drugs in this example; molecular weight of above polyacrylamides polymer was about 8.0×10⁴; target drugs was insulin, which was used with polyacrylamides polymer according to mass ratio of 15:85 in this example; the material of preparing substrate 2 of micro-needle array chip was pure polyacrylamides polymer;

The specific steps were as follows:
1. The mixture of polyacrylamides polymer and insulin was mixed with water according to 20:80 by quality percentage firstly. And it was mixed uniformly by vibration meters. And then the polymer aqueous solution was processed by ultrasonic wave for 5 minutes to remove bubbles;
2. The polymer aqueous solution prepared in step 1, cavity die and horizontal operation platform prepared in examples 4-6 was placed in the glove box; lubricating oil was daubed on the surface of the horizontal operation platform, cavity die was fixed on the surface of the horizontal operation platform;
3. The mixture solution prepared in step 1 was poured into the cavity die as shown in Fig.11 or 12. The volume of mixture solution preparing polymer micro-needle array 1 and thin film of substrate 2 was determined by the volume of chamber 10 of micro-needle array, part of the volume of chamber of substrate 11 and the mass fraction of mixture of polyacrylamides polymer and insulin in mixture solution. The poured polymer aqueous solution was dried, the drying temperature was 40°C; drying the mixture solution comprising polymer and target drugs until it lost liquidity.
4.The mixture comprising polyacrylamides polymer was mixed with water according to 40:60 by mass ratio on the basis of step 1. And then the polymer aqueous solution was processed by ultrasonic wave for 5 minutes to remove bubbles after the polymer dissolved completely;
5.The polymer aqueous solution prepared in step 4 was poured into the cavity die used in step 3; the volume of polymer aqueous solution preparing polymer micro-needle array chip was determined by part of volume of chamber substrate 11, and the mass fraction of polyacrylamides polymer in aqueous solution; the poured polymer aqueous solution was dried, the drying temperature was 40°C; if the polymer aqueous solution in cavity die could not meet the needs of the preparation of substrate 2 of polymer micro-needle array chip, pouring for a second or more times was conducted after parts of the water therein could be dried to remove from the polymer aqueous solution. Then drying and curing the polymer and the target drugs in aqueous solution in the cavity die to obtain the polymer micro-needle array chip as designed.
6. The curing mixture comprising polyacrylamides polymer and insulin was taken out from the cavity die, polymer micro-needle array chip as shown in Fig. 15 were obtained.

Molecular weight of above polyacrylamides polymer was a certain value between 5.0×10⁴ and 2.0×10⁵; it also could be a mixture comprising different molecular weight which was between 5.0×10⁴ and 2.0×10⁵ according to a certain proportion;

The insulin in the mixture of polyacrylamides polymer and insulin was changed in range of 0.1-50 mass%;

The mixture of polyacrylamides polymer and insulin in the mixture aqueous solution was changed in range of 1-80 mass%;

The polyacrylamides polymer in the aqueous solution was changed in range of 30-80 mass%;

The drying temperature could be changed in range of 30-90°C;

The drying duration could be adjusted according to temperature and other conditions.

The insulin was replaced by one or a combination of several component selected from the group consisting of vaccines, polypeptides, proteins, polysaccharides, nucleic acids, hormones, anti-cancer drugs, genetic engineering drugs, natural product drugs, the traditional Chinese medicine or nutrients, of any molecular weight.

### Example 24

This example was used to show the method of preparing polymer micro-needle array chip using cavity die prepared in example 18-20;

Micro-needle bar was prepared by using a mixture of polyacrylamides polymer and target drugs in this example; the target drugs was insulin, which was mixed with polyacrylamides polymer to use according to 10:90 by mass ratio; the material used for preparing substrate 2 of micro-needle array chip was thin film of poly lactic acid;

The specific steps were as follows:
1. The mixture of polyacrylamides polymer and insulin was mixed with water according to 15:85 by mass ratio firstly. And it was mixed uniformly by vibration meters. And then the polymer aqueous solution was processed by ultrasonic wave for 5 minutes to remove bubbles;
2. The mixture solution prepared in step 1, cavity die and horizontal operation platform prepared in examples 4-6 was placed in the glove box; lubricating oil was daubed on the surface of the horizontal operation platform, cavity die was fixed on the surface of the horizontal operation platform;
3. The mixture solution prepared in step 1 was poured into the cavity die as shown in Fig.11 or 12. The volume of mixture solution preparing polymer micro-needle array 1 and substrate 2 film connected with the micro-needle array 1, was determined by the volume of chamber 10 of micro-needle array, part of the volume of chamber of substrate 11 and the mass fraction of mixture of polyacrylamides polymer and insulin in mixture solution. The poured polymer aqueous solution was dried, the drying temperature was 40°C; drying and curing the mixture solution comprising polymer and target drugs completely;
4. The prefabricated thin film of prefabricated polylactic acid was adhered to the back of the substrate of micro-needle array chip;
5. The micro-needle array chip adhering to thin film of prefabricated polylactic acid was taken out from the cavity die, polymer micro-needle array chip as shown in Fig. 15 were obtained.

The above insulin in the mixture of polyacrylamides polymer and insulin was changed in range of 0.1-50 mass%;

Molecular weight of the above polyacrylamides polymer was a certain value between 5.0×10⁴ and 2.0×10⁵; it also could be a mixture comprising different molecular weight which was between 5.0×10⁴ and 2.0×10⁵ according to a certain proportion;

The mixture of polyacrylamides polymer and insulin in the mixture aqueous solution was changed in range of 1-80 mass%;

The drying temperature could be changed in range of 30-90°C;

The drying duration could be adjusted according to temperature and other conditions.

The insulin was replaced by one or a combination of several component selected from the group consisting of vaccines, polypeptides, proteins, polysaccharides, nucleic acids, hormones, anti-cancer drugs, genetic engineering drugs, natural product drugs, the traditional Chinese medicine or nutrients.

### Example 25

This example was used to illustrate the method of preparing a patch for polymer micro-needle transdermal drug delivery using polymer micro-needle array chip prepared in example 21;

The specific steps were as follows:
1. The base plate 3 was prepared to have the same shape as the substrate 2, which has a side length of 7.5 mm and a thickness of 200 µm, using medical plastics, then base plate 3 was adhered to the back of substrate 2;
2. An anti-seepage washer 4 was prepared by natural rubber; the shape of inside of anti-seepage washer 4 was the same as the shape of base plate 3 surrounded by anti-seepage washer 4, whose length of side was 7.5 mm; cross section of anti-seepage washer 4 was rectangular with a height equal to surrounded base plate 3, namely, 200 µm and a width of 500 µm;
3. An anti-seepage layer 7 was combined to the adhesive tape 6 together, and cut into a rectangle with a side length of 25 mm and the width of 10 mm; anti-seepage layer was cotton fiber which was hydrophobic treated;
4. The middle part of anti-adhesion layer 6 with the length of 25 mm and the width of 10 mm was cut, the shape and area of the part cut was the same as the outside of anti-seepage washer 4; the cut anti-adhesion layer 6 was placed around anti-seepage washer 4;
5.The inside of adhesive tape combined with anti-seepage layer 7 was covered on the base plate 3, anti-seepage washer 4 and anti-adhesion layer 5, and then were cohered together.

The prepared micro-needle array 1 of a patch for polymer micro-needle transdermal drug delivery was preserved in the mould, which was used for preparing polymer micro-needle array chip;

The spatial parameter of the above micro-needle array chip, including quantity, height, spacing, thickness of substrate 2, the shortest distance between any position of the very outside of substrate 2 and micro-needle array 1 and the like, could be adjusted according to the needs;

Base plate 12 was made by at least one of material selected from medical polymer, synthetic resin, latex, rubber, glass, ceramics, metal or composite material; structure of the base plate 12 was more films, the more films could be combined each other tightly by cohering, fusion, bonding or physical methods;

The anti-seepage washer 4 was prepared by at least one of material selected from the group consisting of latex, rubber, medical plastics and polymer;

The anti-seepage layer 7 was prepared by at least one of material selected from the group consisting of hydrophobic fiber, latex, rubber and the like.

### Example 26

This example was used to illustrate the method for preparing a patch for micro-needle transdermal drug delivery using polymer micro-needle array chip prepared in example 22;

The method of example 25 was repeated except that material for preparing micro-needle array chip was changed to a mixture of polyacrylamide polymer and target drugs; the target drugs in this example was bovine serum albumin with 20 mass%;

The bovine serum albumin in the mixture of polyacrylamide polymer and bovine serum albumin was changed in range of 0.5-50 mass%;

The bovine serum albumin was replaced by one or a combination of several component selected from the group consisting of arbitrary molecular weight of vaccines, polypeptides, proteins, polysaccharides, nucleic acids, hormones, anti-cancer drugs, genetic engineering drugs, natural product drugs, the traditional Chinese medicine or nutrients.

### Example 27

This example was used to illustrate the method for preparing a patch for micro-needle transdermal drug delivery using polymer micro-needle array chip prepared in example 23;

The method of example 25 was repeated excepted that material for preparing micro-needle array 1 of micro-needle array chip was a mixture of polyacrylamides polymer and target drugs; the target drugs in this example was insulin with 15 mass%;

The above insulin in the mixture of polyacrylamides polymer and insulin was changed in range of 0.1-50 mass%;

The insulin was replaced by one or a combination of several component selected from the group consisting of vaccines, polypeptides, proteins, polysaccharides, nucleic acids, hormones, anti-cancer drugs, genetic engineering drugs, natural product drugs, the ingredient of traditional Chinese medicine or nutrients.

### Example 28

This example was used to illustrate the method for preparing a patch for micro-needle transdermal drug delivery using polymer micro-needle array chip prepared in example 24;

Material for preparing micro-needle array 1 of micro-needle array chip was changed to a mixture of polyacrylamides polymer and target drugs; material prepared for substrate 2 was polylactic acid; the target drugs in this example was insulin with 10 mass%; the back of substrate 2 was not needed to adhere to base plate 12 and was not needed to surround anti-seepage washer 13, when the patch is prepared;

The specific steps were as follows:
1. The middle part of anti-adhesion layer 14 was cut, the shape and area of the part cut was the same as the substrate 2; the cut anti-adhesion layer 14 was placed around substrate 2;
2.The inside of adhesive tape 6 combined with anti-seepage layer 16 covers the substrate 2 and anti-adhesion layer 14, and then were bonded together.

The above insulin in the mixture of polyacrylamides polymer and insulin was changed in range of 0.5-50 mass%;

The insulin was replaced by one or a combination of several component selected from the group consisting of vaccines, polypeptides, proteins, polysaccharides, nucleic acids, hormones, anti-cancer drugs, genetic engineering drugs, natural product drugs, the traditional Chinese medicine or nutrients.

Examples 29-31 was used to illustrate the usage of a patch for polymer micro-needle transdermal drug delivery prepared in examples 25-28;

### Example 29

Freezing pigskin used for simulated human skin was thawed; sites of pesticide application were cleaned and disinfected after thawed;

A patch for micro-needle transdermal drug delivery prepared in example 25 was taken out from mould, and observed under microscope, and found that the number of broken needle was less than or equal to two;

Anti-adhesion layer 14 was peeled off, and micro-needle array was pierced into thawed pigskin by tapping for one minute and then was moved.

Influenza vaccine mixed with fluorescent dye FITC was applied onto the surface of pigskin pierced by micro-needle array, and then was put in the environment that the temperature was not higher than 36°C, and relative humidity was not less than 40% for half an hour;

Pigskin applied with influenza vaccine was observed under microscope, and then found that the surface of pigskin had obvious microporous. Dye could penetrate the skin;

The pesticide applying method was suitable for usage of other vaccines, or less dosage and the dosage of drugs without strict requirements of drugs.

### Example 30

Freezing pigskin used for simulated human skin was thawed; sites of pesticide application were cleaned and disinfected after thawed;

A patch for micro-needle transdermal drug delivery prepared in example 26 was taken out from mould, and observed under microscope, and found that the number of broken needle was less than or equal to two;

Anti-adhesion layer 14 was torn, micro-needle array 1 was pierce into thawed pigskin by tapping and a patch for micro-needle transdermal drug delivery was fixed on the surface of skin by adhesive tape 6; the above pigskin and micro-needle patch were put in the environment that the temperature was not higher than 36°C, and relative humidity was not less than 40% for an hour and then were moved;

Skin removed the micro-needle transdermal patch was observed under the microscope, and found that the surface of skin had obvious microporous. Bovine serum albumin could penetrate the skin;

The pesticide apply method was suitable for usage of cheap, more dosage and the dosage of drugs without strict requirements of drugs.

### Example 31

The method of example 30 was repeated excepted that a patch for micro-needle transdermal drug delivery was changed to a patch for micro-needle transdermal drug delivery prepared in example 27 or 28;

The pesticide apply method was suitable for usage of costly, the dosage of drugs with strict requirements of drugs.

The above detailed description and examples make description in detail for the invention. The description is only for the understanding of the present for one skilled in the art. Various modifications or variations can be made for one skilled in the art based on the present invention. So, all equivalent technical solutions are still within the scope of the present invention. The patent protection of the present invention is defined by the claims.

## Claims

1. A polymer micro-needle array chip, comprising a substrate and a micro-needle array standing thereon, wherein
the material for preparing the micro-needle array is a polyacrylamides polymer;
the molecular weight of the polyacrylamides polymer is in range of 1.0×10⁴-2.0×10⁵;
the Vickers hardness of the polyacrylamides polymer is in range of 150-600 HV;
the impact strength of the polyacrylamides polymer is in range of 5-30 J/m.

2. The polymer micro-needle array chip according to claim 1, wherein a square sheet prepared by the polyacrylamides polymer and having a thickness of 2 mm and sides of length 1 cm, dissolves at least 50 vol% after immersing in a still physiological saline for about 6 hours.

3. The polymer micro-needle array chip according to claim 1 or claim 2, wherein the polyacrylamides polymer is polymerized from acrylamide monomer; the amount of residual acrylamide monomer in the polyacrylamides polymer is not more than 0.5 ppm.

4. The polymer micro-needle array chip according to claim 1, wherein the polyacrylamides polymer is mixed with bioactive substances or drugs, the bioactive substances or drugs being present in the mixture in amount of 0.1-50 mass%; preferably, 10-20 mass%.

5. The polymer micro-needle array chip according to claim 4, wherein the bioactive substances or drugs is one or more selected from the group consisting of vaccines, polypeptides, proteins, polysaccharides, nucleic acids, hormones, anti-cancer drugs, genetic engineering drugs, natural product drugs, traditional Chinese medicine and nutrients.

6. The polymer micro-needle array chip according to any of claims 1 to 5, wherein the micro-needle array comprises at least two micro-needles; the micro-needle comprising a needle head and a needle bar; the needle bar being the body of the micro-needle with one end fixed on the substrate; the needle head being at the top of the micro-needle in any tip-like shape.

7. The polymer micro-needle array chip according to claim 1 or claim 6, wherein the diameter of the largest cross-sectional circle or circumcircle of the micro-needle is in range of 50-1000 µm; the length of the micro-needle is in range of 100-5000 µm; the thickness of the substrate is in range of 50-5000 µm.

8. The polymer micro-needle array chip according to claim 1 or claim 7, wherein the substrate comprises a substrate film and a substrate body; the substrate film connected to the micro-needle array with thickness less than 50 µm.

9. The polymer micro-needle array chip according to claim 1, wherein the substrate is made of polyacrylamides polymer.

10. The polymer micro-needle array chip according to claim 4, wherein the substrate is made of a mixture comprising polyacrylamides polymer and bioactive substances or drugs, the bioactive substances or drugs being present in the mixture in amount of 0.1-50 mass%; preferably, 10-20 mass%.

11. The polymer micro-needle array chip according to claim 8, wherein the micro-needle array and of the substrate film of the polymer micro-needle array chip is made of a mixture comprising the polyacrylamides polymer and bioactive substances or drugs; the bioactive substances or drugs being present in the mixture in amount of 0.1-50 mass%, preferably, 10-20 mass%; and the substrate body is made of polyacrylamides polymer.

12. The polymer micro-needle array chip according to claim 8, wherein the micro-needle array and the substrate film is made of polyacrylamides polymer, and the substrate body is a combination of one or more layers selected from the group consisting of polylactic acid, polyethylene, polypropylene, poly(butylene succinate), rubber, latex, glass and metal thermoplastic composite materials, respectively.

13. The polymer micro-needle array chip according to claim 8, wherein the micro-needle array and the substrate film is made of a mixture comprising the polyacrylamides polymer and the bioactive substances or drugs; the bioactive substances or drugs being present in the mixture in amount of 0.1-50 mass%, preferably, 10-20 mass%; and the substrate body is a combination of one or more layers selected from the group consisting of polylactic acid, polyethylene, polypropylene, poly(butylene succinate), rubber, latex, glass and metal thermoplastic composite materials, respectively.

14. The polymer micro-needle array chip according to any of claims 1, 2, 4, and 9-13, wherein a method for preparing the polyacrylamides polymer comprising:
a synthetic reaction system comprising alcohols-based organic solvent, water, acrylamide monomer and initiator; introducing the high purity nitrogen continuously into the reaction system , stirring rising the temperature to and holding at the target temperature, during the reaction; removing the acrylamide monomer from the reaction product and drying to obtain the polyacrylamides polymer, after the reaction.

15. The polymer micro-needle array chip according to claim 14, wherein the method for preparing the polyacrylamides polymer comprising the steps of:
S-1, adding an organic solvent, water and acrylamide monomer in prescribed amounts into a reactor equipped with a stirring device;
the organic solvent comprising an alcoholic solvent and a ketonic solvent; the alcoholic solvent being one or more selected from the group consisting of methanol, ethanol, n-propanol, isopropanol, n-butanol; the alcoholic solvent being present in the reaction system in amount of not less than 60 vol%; the ketonic organic solvent being one or more selected from the group consisting of acetone, butanone, methyl isobutyl ketone, cyclohexanone; the ketonic solvent being present in the reaction system in amount of not more than 25 vol%;
water being present in the reaction system in amount of not more than 25 vol%;
the initial concentration of the acrylamide monomer in the reaction system being in range of 0.1-3 mol/L;
S-2, introducing the high purity nitrogen into the reactor of reaction system comprising the solvent, water and acrylamide monomer to remove oxygen, stirring, and rising the temperature of the reaction system up to the target temperature in range of 30-85°C;
S-3, adding the initiator into the reaction system when the temperature reaches the target temperature, with stirring and introducing the nitrogen; the initiator being an azo initiator or peroxide; the azo initiator being one or more selected from a group consisting of 2,2-azobisisobutyronitrile, 2,2'-azobisisoheptonitrile, 2,2'-azobis[2-methylpropionamidine]dihydrochloride, diisopropyl 2,2'-azobisbutyrate, dimethyl 2,2'-azobis(2-methylpropionate); the peroxide being one or more selected from the group consisting of ammonium persulfate, sodium persulfate, potassium persulfate, tertiary butyl peroxide, dicumyl peroxide and benzoyl peroxide; preferably, the initiator further comprising a reducing agent; more preferably, the reducing agent being one or two selected from a group consisting of sodium bisulfite or sodium metabisulfite; the amount of the initiator being in range of 0.01-1 wt% of the acrylamide monomer;
S-4, after the addition of the initiator, keeping the target temperature for 8-30 hours with stirring and introducing the nitrogen; the target temperature being in range of 30-85°C;
S-5, after the reaction, vacuum filtering a solid-liquid mixture in the reactor, then drying the resultant solid product at the temperature in range of 30-70°C;
S-6, dissolving the resultant dry product in water completely, adding the organic solvent which dissolves only the acrylamide monomer not the polymerization product to re-precipitate for removing the unreacted acrylamide monomer; the organic solvent being one or more selected from the group consisting of methanol, ethanol, n-propanol, isopropanol, n-butanol, acetone, butanone, methyl isobutyl ketone and cyclohexanone;
repeating the steps of S-5 and S-6 for 2-4 times;
S-7, drying the resultant product with the acrylamide monomer removed in a vacuum oven at the temperature in range of 30-70°C for 20-50 hours to obtain the polyacrylamides polymer.

16. A method for preparing the polymer micro-needle array chip according to claim 9, wherein comprising steps of:
1) preparing a prototype of micro-needle array chip, which has the same spatial features as that of the target polymer micro-needle array chip, from one or more metal material selected from the group consisting of titanium, copper, aluminum, nickel, tungsten, stainless steel, titanium alloy, nickel alloy, aluminum alloy and copper alloy;
2) preparing a cavity die by using the resultant prototype of metal micro-needle array chip and one or more polymer material selected from the group consisting of polyethylene, polypropylene, polylactic acid, poly(butylene succinate) and polydimethylsiloxane;
3) removing the prototype of micro-needle array chip from the cavity die;
4) mixing the polyacrylamides polymer with water to obtain an aqueous solution at the temperature in range of 10-90°C; the polyacrylamides polymer being present in the aqueous solution in amount of 1-80 mass%, preferably, 10-50 mass%;
5) ultrasonic processing the aqueous solution prepared in step 4) to remove bubbles;
6) pouring the aqueous solution prepared in step 5) into the cleaned cavity die, and placing the cavity die with the aqueous solution onto a horizontal operation platform; preferably, placing the cavity die and the horizontal operation platform in a closed system; preferably, sealing the horizontal operation platform and the cavity die with a viscous liquid;
7) drying the aqueous solution poured into the cavity die in step 6) at the temperature in range of 20-90°C to cure the polyacrylamides polymer of the aqueous solution in the cavity die and obtain the polymer micro-needle array chip.

17. The method according to claim 16, wherein the step 6) and step 7) further comprising the steps of:
A1, in case that the polyacrylamide polymer of the aqueous solution for a once-through pour in the cavity die is sufficient to prepare the polymer micro-needle array chip, drying the aqueous solution in the cavity die directly and curing the polyacrylamides polymer therein to obtain the polymer micro-needle array chip;
B1, in case that the polyacrylamide polymer of the aqueous solution for a once-through pour in the cavity die is not sufficient to prepare the polymer micro-needle array chip, drying the aqueous solution in the cavity die to remove part of the water therein; pouring for a second time or more times until the polyacrylamide polymer in the cavity die is sufficient to prepare the polymer micro-needle array chip; drying the aqueous solution and curing the polyacrylamides polymer to obtain the polymer micro-needle array chip.

18. A method for preparing the polymer micro-needle array chip according to claim 10, wherein comprising steps of:
1) preparing a prototype of micro-needle array chip, which has the same spatial features as that of the target polymer micro-needle array chip, from one or more metal material selected from the group consisting of titanium, copper, aluminum, nickel, tungsten, stainless steel, titanium alloy, nickel alloy, aluminum alloy and copper alloy;
2) preparing a cavity die by using the resultant prototype of metal micro-needle array chip and one or more polymer material selected from the group consisting of polyethylene, polypropylene, polylactic acid, poly(butylene succinate) and polydimethylsiloxane;
3) removing the prototype of micro-needle array chip from the cavity die;
4) mixing the polyacrylamides polymer with the bioactive substances or drugs to obtain a mixture; the bioactive substances or drugs being present in the mixture in amount of 0.1-50 mass%, preferably, 10-20 mass%;
5) mixing the mixture prepared in step 4) with water to obtain the mixture solution at the temperature in range of 10-90°C; the mixture being present in the mixture solution in amount of 1-80 mass%, preferably, 10-50 mass%;
6) ultrasonic processing the mixture solution prepared in step 5) to remove bubbles;
7) pouring the mixture solution prepared in step 6) into the cleaned cavity die, and placing the cavity die with the mixture solution onto a horizontal operation platform; preferably, placing the cavity die and the horizontal operation platform in a closed system; preferably, sealing the horizontal operation platform and the cavity die with a viscous liquid;
8) drying the mixture solution poured into the cavity die in step 7) at the temperature in range of 20-90°C to cure the mixture of polyacrylamides polymer and bioactive substances or drugs in the mixture solution in the cavity die to obtain the polymer micro-needle array chip.

19. The method according to claim 18, wherein step 7) and step 8) further comprising the steps of:
A2, in case that the mixture of the mixture solution for a once-through pour in the cavity die is sufficient to prepare the polymer micro-needle array chip, drying the mixture solution in the cavity die directly and curing the mixture to obtain the polymer micro-needle array chip;
B2, in case that the mixture of the mixture solution for a once-through pour in the cavity die is not sufficient to prepare the polymer micro-needle array chip, drying the mixture solution in the cavity die to remove part of the water therein; pouring for a second time or more times until the mixture in the cavity die is sufficient to prepare the polymer micro-needle array chip; drying the mixture solution and curing the mixture to obtain the polymer micro-needle array chip.

20. A method for preparing the polymer micro-needle array chip according to claim 11, wherein comprising the following steps:
1) preparing a prototype of micro-needle array chip, which has the same spatial features as that of the target polymer micro-needle array chip, from one or more metal material selected from the group consisting of titanium, copper, aluminum, nickel, tungsten, stainless steel, titanium alloy, nickel alloy, aluminum alloy and copper alloy;
2) preparing a cavity die by using the resultant prototype of metal micro-needle array chip and one or more polymer material selected from the group consisting of polyethylene, polypropylene, polylactic acid, poly(butylene succinate) and polydimethylsiloxane;
3) removing the prototype of micro-needle array chip from the cavity die;
4) mixing the polyacrylamides polymer with the bioactive substances or drugs to obtain a mixture; the bioactive substances or drugs being present in the mixture in amount of 0.1-50 mass%, preferably, 10-20 mass%;
5) mixing the mixture prepared in step 4) with water to obtain the mixture solution at the temperature in range of 10-90°C; the mixture being present in the mixture solution in amount of 1-80 mass%, preferably, 10-50 mass%;
6) ultrasonic processing the mixture solution prepared in step 5) to remove bubbles;
7) pouring the mixture solution prepared in step 6) into the cleaned cavity die, and placing the cavity die with the mixture solution onto a horizontal operation platform; preferably, placing the cavity die and the horizontal operation platform in a closed system; preferably, sealing the horizontal operation platform and the cavity die with a viscous liquid;
8) drying the mixture solution poured into the cavity die in step 7) at the temperature in range of 20-90°C to cure the mixture of polyacrylamides polymer and bioactive substances or drugs in the mixture solution in the cavity die to obtain the micro-needle array and the substrate film connected thereto with a thickness of less than 50 µm, of the polymer micro-needle array chip;
9) mixing the polyacrylamides polymer with water to obtain the aqueous solution at the temperature in range of 10-90°C; the polyacrylamides polymer being present in the aqueous solution in amount of 20-80 mass%; preferably, 30-50 mass%; preferably, the polyacrylamides polymer in once-through pour of the aqueous solution in the cavity die is sufficient to prepare the substrate of the polymer micro-needle array chip;
10) ultrasonic processing the mixture solution prepared in step 9) to remove bubbles;
11) pouring the aqueous solution prepared in step 10) into the cavity die in step 7) and step 8) continually;
12) drying the aqueous solution poured into the cavity die in step 11) at the temperature in range of 20-90°C to cure the polyacrylamides polymer in the aqueous solution in the cavity die to obtain the entire polymer micro-needle array chip.

21. The method according to claim 20, wherein step 7) and step 8) further comprising the steps of:
A3, in case that the mixture of the mixture solution for a once-through pour in the cavity die is sufficient to prepare the micro-needle array and the substrate film of the polymer micro-needle array chip, drying the mixture solution in the cavity die directly and curing the mixture to obtain the micro-needle array and the substrate film of the polymer micro-needle array chip;
B3, in case that the mixture of the mixture solution for a once-through pour in the cavity die is not sufficient to prepare the micro-needle array and the substrate film of the polymer micro-needle array chip, drying the mixture solution in the cavity die to remove part of the water therein; pouring for a second time or more times until the mixture in the cavity die is sufficient to prepare the micro-needle array and the substrate film of the polymer micro-needle array chip; drying the mixture solution and curing the mixture to obtain the micro-needle array and the substrate film of the polymer micro-needle array chip, as designed.

22. A method for preparing the polymer micro-needle array chip according to claim 12, wherein comprising the steps of:
1) preparing a prototype of micro-needle array chip, which has the same spatial features as that of the target polymer micro-needle array chip, from one or more metal material selected from the group consisting of titanium, copper, aluminum, nickel, tungsten, stainless steel, titanium alloy, nickel alloy, aluminum alloy and copper alloy;
2) preparing a cavity die by using the resultant prototype of metal micro-needle array chip and one or more polymer material selected from the group consisting of polyethylene, polypropylene, polylactic acid, poly(butylene succinate) and polydimethylsiloxane;
3) removing the prototype of micro-needle array chip from the cavity die;
4) mixing the polyacrylamide polymer with water to obtain an aqueous solution at the temperature in range of 10-90°C; the polyacrylamide polymer being present in the aqueous solution in amount of 1-80 mass%, preferably, 10-50 mass%;
5) ultrasonic processing the aqueous solution prepared in step 4) to remove bubbles;
6) pouring the aqueous solution prepared in step 5) into the cleaned cavity die, and placing the cavity die with the aqueous solution onto a horizontal operation platform; preferably, placing the cavity die and the horizontal operation platform in a closed system; preferably, sealing the horizontal operation platform and the cavity die with a viscous liquid;
7) drying the aqueous solution poured into the cavity die in step 6) at the temperature in range of 20-90°C to cure the polyacrylamides polymer of the aqueous solution in the cavity die and obtain the micro-needle array and the substrate film connected thereto, of the polymer micro-needle array chip;
8) connecting the micro-needle array and the substrate film of the polymer micro-needle array chip prepared in step 7) with the substrate body made of one or more film to obtain the polymer micro-needle array chip; the one or more film is made of one or more material selected from the group consisting of polyethylene, polypropylene, poly(butylene succinate), polydimethylsiloxane, rubber, polylactic acid, latex, glass and metal thermoplastic composite materials; the substrate body made of the one or more film is combined tightly with the substrate film by cohering, fusion and bonding.

23. The method according to claim 22, wherein step 6) and step 7) comprise the steps of:
F4, in case that the polyacrylamides polymer in aqueous solution for a once-through pour in the cavity die is sufficient to prepare the micro-needle array and the substrate film of the polymer micro-needle array chip, drying the aqueous solution in the cavity die directly and curing the polyacrylamides polymer to obtain the micro-needle array and the substrate film of the polymer micro-needle array chip;
G4, in case that the polyacrylamides polymer in aqueous solution for a once-through pour in the cavity die is not sufficient to prepare the micro-needle array and the substrate film of the polymer micro-needle array chip, drying the aqueous solution in the cavity die to remove part of the water therein; pouring for a second time or more times until the polyacrylamides polymer in the cavity die is sufficient to prepare the micro-needle array and the substrate film of the polymer micro-needle array chip; drying the aqueous solution and curing the polyacrylamides polymer to obtain the micro-needle array and the substrate film of the polymer micro-needle array chip.

24. A method for preparing the polymer micro-needle array chip according to claim 13, wherein comprising the steps of:
1) preparing a prototype of micro-needle array chip, which has the same spatial features as that of the target polymer micro-needle array chip, from one or more metal material selected from the group consisting of titanium, copper, aluminum, nickel, tungsten, stainless steel, titanium alloy, nickel alloy, aluminum alloy and copper alloy;
2) preparing a cavity die by using the resultant prototype of metal micro-needle array chip and one or more polymer material selected from the group consisting of polyethylene, polypropylene, polylactic acid, poly(butylene succinate) and polydimethylsiloxane;
3) removing the prototype of micro-needle array chip from the cavity die;
4) mixing the polyacrylamides polymer with the bioactive substances or drugs to obtain a mixture; the bioactive substances or drugs being present in the mixture in amount of 0.1-50 mass%, preferably, 10-20 mass%;
5) mixing the mixture prepared in step 4) with water to obtain the mixture solution at the temperature in range of 10-90°C; the mixture being present in the in amount of 1-80 mass%, preferably, 10-50 mass%;
6) ultrasonic processing the mixture solution prepared in step 5) to remove bubbles;
7) pouring the mixture solution prepared in step 6) into the cleaned cavity die, and placing the cavity die with the mixture solution onto a horizontal operation platform; preferably, placing the cavity die and the horizontal operation platform in a closed system; preferably, sealing the horizontal operation platform and the cavity die with a viscous liquid;
8) drying the mixture solution poured into the cavity die in step 7) at the temperature in range of 20-90°C to cure the mixture of polyacrylamides polymer and bioactive substances or drugs in the mixture solution in the cavity die to obtain the micro-needle array and the substrate film of the polymer micro-needle array chip;
9) connecting the micro-needle array and the substrate film of the polymer micro-needle array chip prepared in step 7) with the substrate body made of one or more film to obtain the polymer micro-needle array chip; the one or more film is made of one or more material selected from the group consisting of polyethylene, polypropylene, poly(butylene succinate), polydimethylsiloxane, rubber, polylactic acid, latex, glass and metal thermoplastic composite materials; the substrate body made of the one or more film is combined tightly with the substrate film by cohering, fusion and bonding

25. The method according to claim 24, wherein step 7) and step 8) comprising the steps of:
A5, in case that the mixture of the mixture solution for a once-through pour in the cavity die is sufficient to prepare the micro-needle array and the substrate film of the polymer micro-needle array chip, drying the mixture solution in the cavity die directly and curing the mixture to obtain the micro-needle array and the substrate film of the polymer micro-needle array chip;
B5, in case that the mixture of the mixture solution for a once-through pour in the cavity die is not sufficient to prepare the micro-needle array and the substrate film of the polymer micro-needle array chip, drying the mixture solution in the cavity die to remove part of the water therein; pouring for a second time or more times until the mixture in the cavity die is sufficient to prepare the micro-needle array and the substrate film of the polymer micro-needle array chip; drying the mixture solution and curing the mixture to obtain the micro-needle array and the substrate film of the polymer micro-needle array chip, as designed.

26. A patch for micro-needle transdermal drug delivery, wherein comprising a polymer micro-needle array chip according to any of claims 9 to 13, a base plate, an anti-seepage washer, an anti-adhesion layer, an adhesive tape and an anti-seepage layer; the polymer micro-needle array chip comprises a substrate and a micro-needle array standing thereon; the base plate being one or more film adhered to the back of the substrate of the polymer micro-needle array chip; the anti-seepage washer is a layer of washer surrounding the edge of the substrate of the polymer micro-needle array chip and the base plate; the anti-adhesion layer covers the area outside the anti-seepage washer; the adhesive tape is a double sided sticky tape, one side of the tape covers the base plate, the anti-seepage washer and the anti-adhesion layer, the other side of the tape is adhered to the anti-seepage layer, which is a protective film.

27. A method for preparing a patch for micro-needle transdermal drug delivery according to claim 26, wherein comprising the steps of:
1) adhering the base plate to the substrate of the polymer micro-needle array chip;
2) fixing the anti-seepage washer around the base plate of the micro-needle patch;
3) placing the anti-adhesion layer at the outside of the micro-needle base plate or that of the substrate of the polymer micro-needle array chip;
4) adhering one side of the double sided sticky tape to the base plate, the anti-seepage washer and the anti-adhesion layer of the micro-needle patch;
5) adhering the anti-seepage layer to the other side of the adhesive tape so as to obtain the patch for polymer transdermal drug delivery.
